# FASCICULE DE BREVET EUROPEEN

(11) **EP 1 106 608 B1**
(45) Date de publication et mention de la délivrance du brevet: **05.03.2003**
(21) Numéro de dépôt: 00403399.9
(22) Date de dépôt: 05.12.2000
(51) Int. Cl.: C07D 265/36, C07D 279/16, C07D 413/12, A61K 31/538, A61K 31/5415, A61P 25/00

(54) **Nouveaux dérivés (dihydro)benzoxaziniques et (dihydro)benzothiaziniques substitués, leur procédé de préparation et les compositions pharmaceutiques qui les contiennent**
Substituierte (Dihydro)benzoxazin- und (Dihydro)benzothiazinderivate, Verfahren zu deren Herstellung und sie enthaltende pharmazeutische Zusammensetzungen
Substituted (dihydro)benzoxazine and (dihydro)benzothiazine derivatives, process for their preparation and pharmaceutical compositions containing them

(30) Priorité: 06.12.1999 FR 9915309
(43) Date de publication de la demande: 13.06.2001
(73) Titulaire: LES LABORATOIRES SERVIER, 92200 Neuilly sur Seine (FR)
(72) Inventeur: Viaud, Marie-Claude, 37000 Tours (FR); Guillaumet, Gérald, 45650 Saint Jean le Blanc (FR); Daubos, Philippe, 28240 La Loupe (FR); Bennejean, Caroline, 94220 Charenton le Pont (FR); Delagrange, Philippe, 92130 Issy les Moulineaux (FR); Renard, Pierre, 78150 Le Chesnay (FR)

(56) Documents cités:
- EP-A- 0 441 539
- WO-A-99/50257
- FR-M- 7 697

## Description

La présente invention concerne de nouveaux dérivés (dihydro)benzoxaziniques et (dihydro)benzothiaziniques substitués, leur procédé de préparation et les compositions pharmaceutiques qui les contiennent.

On trouve dans la littérature de nombreuses structures benzoxaziniques et benzothiaziniques substituées utiles tant en synthèse (Tetrahedron, 53(26), 1997, pp. 8853-8870 ; Heterocycl. Commun., 2 (3), 1996, pp. 273-274 ; J. Chem. Soc., Perkin Trans. 1, (10), 1991, pp. 2525-2529; Chem. Pharm. Bull., 34 (1), 1986, pp. 130-139; Indian J. Pharm., 35 (2), 1973, pp. 58-59) qu'en tant que modulateurs des canaux potassiques (Eur. J. Med. Chem., 33 (12), 1998, pp. 957-967 ; Chem. Pharm. Bull., 44 (1), 1996, pp. 103-114), ou encore en tant qu'agents anticancéreux (Heterocycl. Commun., 3 (3), 1997, pp. 279-284 ; Heterocycl. Commun., 2 (6), 1996, pp. 587-592 ; Anti-Cancer Drugs, 6 (5), 1995, pp. 693-696).

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272), analgésiques (Pharmacopsychiat., 1987, 20, pp. 222-223), ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p. 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sites de liaisons mélatoninergiques.

Plus particulièrement, la présente invention concerne les composés de formule (I): 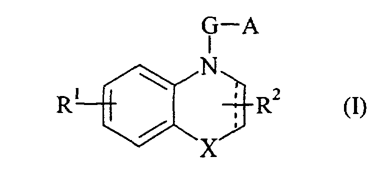 dans laquelle :
◆ R¹ représente un atome d'halogène ou un groupement R, OR, SR, SO₂NRR', -NRR', 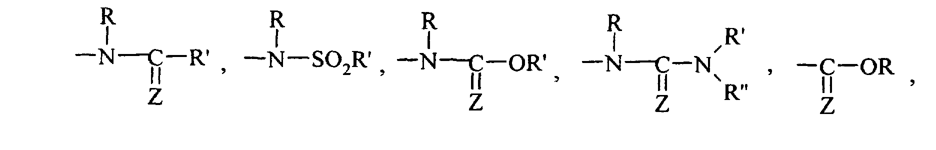 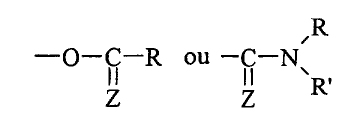 (où Z représente un atome de soufre ou d'oxygène et R, R' et R", identiques ou différents, représentent un atome d'hydrogène ou un groupement alkyle (C₁-C₆) linéaire ou ramifié substitué ou non substitué, alkényle (C₂-C₆) linéaire ou ramifié substitué ou non substitué, alkynyle (C₂-C₆) linéaire ou ramifié substitué ou non substitué, cycloalkyle (C₃-C₈) substitué ou non substitué, cycloalkyle (C₃-C₈) alkyle (C₁-C₆) linéaire ou ramifié substitué ou non substitué, aryle, arylalkyle (C₁-C₆) linéaire ou ramifié, hétéroaryle ou hétéroarylalkyle (C₁-C₆) linéaire ou ramifié,
   (R et R') ou (R' et R") pouvant former ensemble, avec l'atome d'azote qui les porte un groupement morpholinyle, pipéridinyle, pipérazinyle ou pirrolidinyle),
◆ G représente un groupement (CH₂)ₙ dans lequel n vaut 2 ou 3,
◆ A représente un groupement 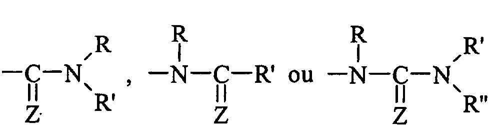 (où R, R', R" et Z sont tels que définis précédemment),
◆ R² représente un atome d'halogène ou un groupement R, OR, COR, COOR ou OCOR (où R est tel que défini précédemment),
◆ X représente un atome d'oxygène ou de soufre,
◆ la notation ---- signifie que la liaison est simple ou double, étant entendu que la valence des atomes est respectée,
étant entendu que :
- sauf précision contraire, les groupements comportant une partie alkyle dans leur structure contiennent de 1 à 6 atomes de carbone et peuvent être linéaires ou ramifiés,
- le terme « substitué » affecté aux expression « alkyle », « alkényle », « alkynyle », « cycloalkyle » signifie que ces groupements peuvent être substitués par un ou plusieurs groupements, identiques ou différents, choisis parmi hydroxy, alkoxy, alkyle, polyhalogénoalkyle, amino (non substitué ou substitué par un ou deux groupements alkyle (C₁-C₆) linéaire ou ramifié) et atomes d'halogène,
- le terme « substitué » affecté à l'expression « cycloalkylalkyle » signifie que la partie cyclique du groupement est substituée par un ou plusieurs groupements, identiques ou différents, choisis parmi hydroxy, alkoxy, alkyle, polyhalogénoalkyle, amino (non substitué ou substitué par un ou deux groupements alkyle(C₁-C₆)linéaire ou ramifié) et atomes d'halogène,
- par «aryle » on entend un groupement phényle, naphtyle ou biphényle, ces groupements étant non substitués ou substitués par un ou plusieurs groupements, identiques ou différents, choisis parmi hydroxy, alkoxy, alkyle, amino, alkylamino, dialkylamino, nitro, cyano, polyhalogénoalkyle, formyle, carboxy, alkyloxycarbonyle, amido, et atomes d'halogène,
- par « hétéroaryle » on entend tout groupement aromatique mono ou bi-cyclique, contenant un à trois hétéroatomes choisis parmi oxygène, soufre et azote, ces groupements étant non substitués ou substitués par un ou plusieurs groupements, identiques ou différents, choisis parmi hydroxy, alkoxy, alkyle, amino, alkylamino, dialkylamino, nitro, cyano, polyhalogénoalkyle, formyle, carboxy, alkyloxycarbonyle, amido, et atomes d'halogène,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels X représente un atome d'oxygène.

Plus particulièrement, l'invention concerne les dérivés benzoxaziniques, et plus préférentiellement les composés dihydrobenzoxaziniques.

Les substituants R¹ préférés sont les groupements alkyle, alkoxy ou hydroxy.

Les substituants R² préférés sont l'atome d'hydrogène ou les groupements aryle, hétéroaryle, arylalkyle ou hétéroarylalkyle, et plus particulièrement le groupement phényle substitué ou non.

Les substituants A préférés sont les groupements NHCOR et CONHR.

Encore plus particulièrement, l'invention concerne les composés de formule (I) qui sont :
* le *N*-[2-(6-hydroxy-2,3-dihydro-4*H*-1,4-benzoxazin-4-yl)éthyl]acétamide,
* le *N*-[2-(6-méthoxy-2,3-dihydro-4*H*-1,4-benzoxazin-4-yl)éthyl]acétamide,
* le *N*-[2-(6-méthoxy-2,3-dihydro-4*H*-1,4-benzoxazin-4-yl)éthyl]butanamide,
* le *N*-[2-(6-méthoxy-2,3-dihydro-4*H*-1,4-benzoxazin-4-yl)éthyl]-3-buténamide,
* le *N-*[2-(6-méthoxy-2,3-dihydro-4*H*-1,4-benzoxazin-4-yl)éthyl]cyclopropanecarboxamide,
* le *N*-[2-(6-méthoxy-2,3-dihydro-4*H*-1,4-benzoxazin-4-yl)éthyl]-2-furamide,
* le *N*-[2-(6-méthoxy-2,3-dihydro-4*H*-1,4-benzoxazin-4-yl)éthyl]benzamide,
* le *N*-[2-(6-hydroxy-2,3-dihydro-4*H*-1,4-benzoxazin-4-yl)éthyl]-2-furamide,
* le *N*-[2-(6-méthoxy-3-phényl-2,3-dihydro-4*H*-1,4-benzoxazin-4-yl)éthyl]acétamide,
* le *N*-[2-(6-méthoxy-3-phényl-2,3-dihydro-4*H*-1,4-benzoxazin-4-yl)éthyl]benzamide,
* le *N*-[2-(6-méthoxy-3-phényl-2,3-dihydro-4*H*-1,4-benzoxazin-4-yl)éthyl]-2-furamide,
* le *N*-[2-(6-méthoxy-3-phényl-2,3-dihydro-4*H*-1,4-benzoxazin-4-yl)éthyl]butanamide,
* le *N*-[2-(6-méthoxy-2-phényl-2,3-dihydro-4*H*-1,4-benzoxazin-4-yl)éthyl]acétamide,
* le *N*-[2-(6-méthoxy-2-phényl-2,3-dihydro-4*H*-1,4-benzoxazin-4-yl)éthyl]-2-furamide,
* le *N*-[2-(6-méthoxy-2-phényl-2,3-dihydro-4*H*-1,4-benzoxazin-4-yl)éthyl]benzamide,
* le *N*-[2-(6-méthoxy-2-phényl-2,3-dihydro-4*H*-1,4-benzoxazin-4-yl)éthyl]butanamide,
* le *N*-[2-(6-méthoxy-2-phényl-2,3-dihydro-4*H*-1,4-benzoxazin-4-yl)éthyl]cyclopropane carboxamide.

Les énantiomères, diastéroisomères ainsi que les sels d'addition à un acide ou à une base pharmaceutiquement acceptable des composés préférés de l'invention font partir intégrante de l'invention.

L'invention s'étend également au procédé de préparation des composés de formule (I) caractérisé en ce que l'on utilise comme produit de départ le composé de formule (II): 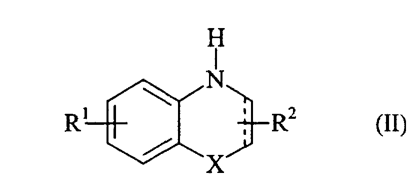 dans laquelle R¹, R², X et la représentation ---- sont tels que définis dans la formule (I),
sur lequel on fait réagir, en milieu basique, le composé de formule (III):

Br-G-CN (III)

dans laquelle G est tel que défini dans la formule (I),
pour conduire au composé de formule (IV): 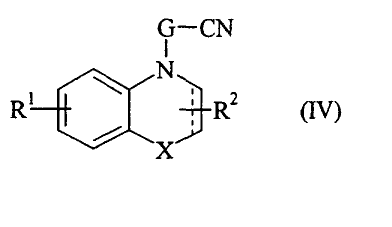 dans laquelle R¹, R², X, G et la représentation ---- sont tels que définis précédemment,
que l'on soumet à une hydrolyse, dans des conditions acides ou basiques afin de conduire au composé de formule (V): 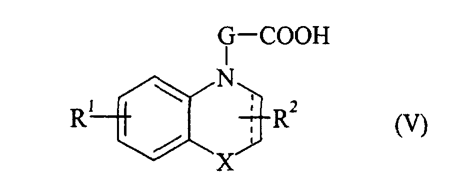 dans laquelle R¹, R², X, G et la représentation ---- ont la même définition que précédemment,
sur lequel on fait agir, en présence d'un agent de couplage ou après transformation en chlorure d'acide correspondant, une amine HNRR' où R et R' sont tels que définis dans la formule (I), pour conduire au composé de formule (I/a), cas particulier des composés de formule (I): 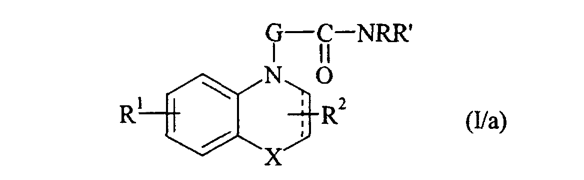 dans laquelle R¹, R², X, G, R, R' et la représentation ---- sont tels que définis précédemment,
composé de formule (I/a) qui est soumis, dans le cas où R et R' représentent simultanément un atome d'hydrogène, à l'action de NaOBr afin de conduire, après hydrolyse, au composé de formule (VI): 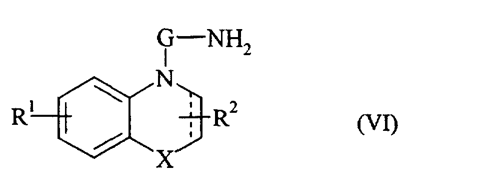 dans laquelle R¹, R², X, G et la représentation ---- sont définis comme précédemment,
composés de formule (VI) (pouvant par ailleurs être obtenus par réduction du composé de formule (IV)) que l'on soumet à l'action :
- d'un chlorure d'acyle de formule (VII) : 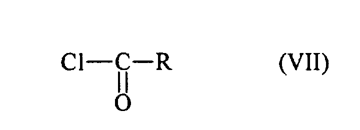 où R est tel que défini précédemment, ou l'anhydride d'acide (mixte ou symétrique) correspondant,
   pour obtenir le composé de formule (I/b), cas particulier des composés de formule (I): 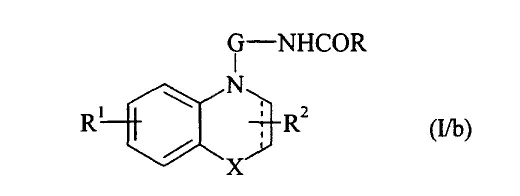 dans laquelle R¹, R², R, X, G et la représentation ---- ont la même définition que précédemment,
- ou d'un composé de formule (VIII):

   O = C = N - R (VIII)

   dans laquelle R est tel que défini précédemment,
   pour conduire au composé de formule (I/c), cas particulier des composés de formule (I): 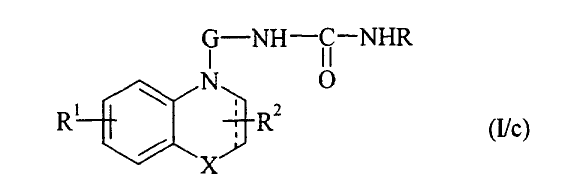 dans laquelle R¹, R², R, X, G et la représentation ---- sont définis comme précédemment,
les composés de formules (I/b) et (I/c) pouvant être soumis à l'action d'un composé de formule (IX):

Rₐ -J (IX)

dans laquelle Rₐ peut prendre toutes les valeurs de R à l'exception de l'atome d'hydrogène et J représente un groupe partant comme un atome d'halogène ou un groupement tosyle,
pour conduire au composé de formule (I/d), cas particulier des composés de formule (I): 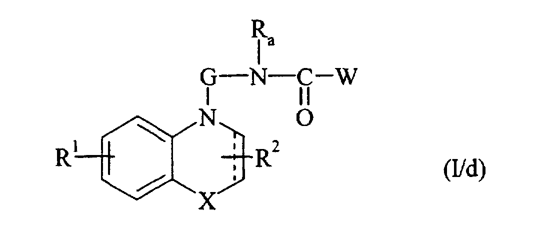 dans laquelle R¹, R², X, G, Rₐ et la représentation ---- ont la même définition que précédemment et W représente un groupement R ou -NRR' où R et R' sont tels que définis précédemment,
et/ou les composés de formules (I/a), (I/b), (I/c) et (I/d) peuvent être soumis à l'action d'un agent de thionation, comme le réactif de Lawesson pour conduire au composé de formule (I/e), cas particulier des composés de formule (I): 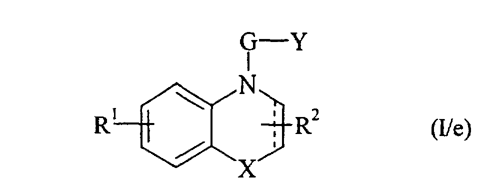 dans laquelle R¹, R², X, G et la représentation ---- sont définis comme précédemment et Y représente un groupement 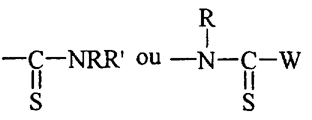 dans lesquels R, R' et W sont tels que définis précédemment,
les composés (I/a) à (I/e) formant l'ensemble des composés de formule (I) et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

Les composés de formule (II) sont soit commerciaux, soit accessibles à l'homme du métier par des réactions chimiques classiques.

En particulier, les composés de formule (II) peuvent être obtenus à partir des composés de formule (X): 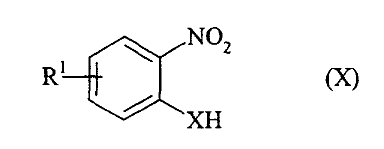 dans laquelle R¹ et X sont tels que définis précédemment,
que l'on soumet à une hydrogénation catalytique pour obtenir le composé de formule (XI): 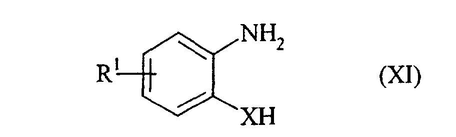 dans laquelle R¹ et X sont définis comme précédemment,
qui est acétylé pour conduire au composé de formule (XII): 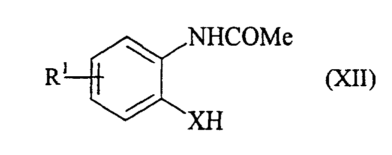 dans laquelle R¹ et X sont tels que définis précédemment,
sur lequel on fait agir un composé de formule (XIII): 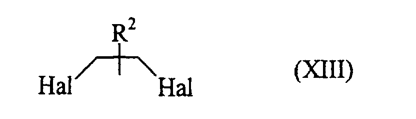 dans laquelle Hal représente un atome d'halogène et R² est tel que défini précédemment,
pour conduire au composé de formule (XIV): 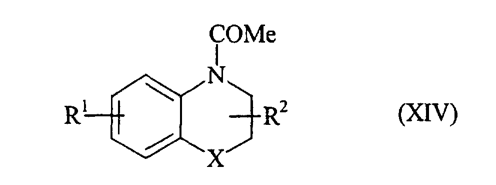 dans laquelle R¹, R² et X sont définis comme précédemment,
qui est hydrolysé en milieu basique afin d'obtenir le composé de formule (XV), cas particulier des composés de formule (II): 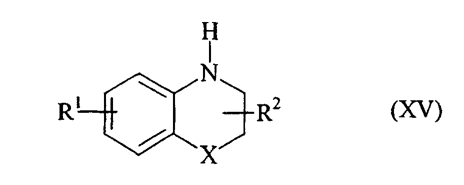 dans laquelle R¹, R² et X sont définis comme précédemment,
composé de formule (XV) pouvant également être obtenu à partir du composé de formule (X) que l'on soumet à l'action d'un dérivé de formule (XVI): 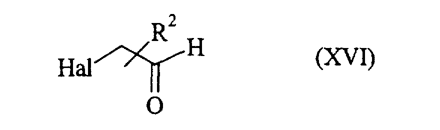 dans laquelle Hal représente un atome d'halogène et R² est tel que défini précédemment,
pour conduire au composé de formule (XVII): 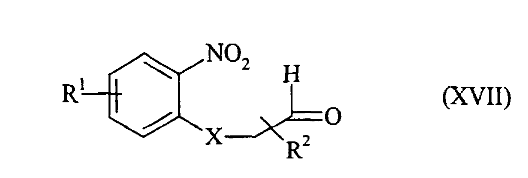 dans laquelle R¹, X et R² sont tels que définis précédemment,
qui est soumis à une hydrogénation catalytique pour conduire au composé de formule (XV), cas particulier des composés de formule (II),
composé de formule (XV) pouvant être par ailleurs obtenu, lorsque le groupement R² est en ortho de X, à partir du composé de formule (XI) que l'on soumet à l'action d'un composé de formule (XVIII) en milieu basique : 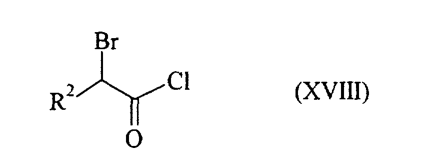 dans laquelle R² est tel que défini précédemment,
puis à l'action de K₂CO₃ pour conduire au composé de formule (XIX): 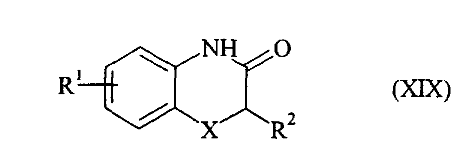 dans laquelle R¹, R² et X sont définis comme précédemment,
qui est soumis à un agent de réduction comme LiAlH₄ par exemple, pour obtenir le composé de formule (XV'), cas particulier des composés de formule (XV): 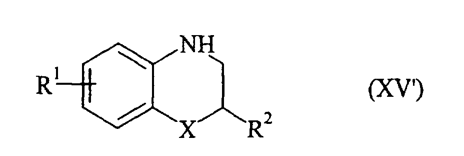 dans laquelle R¹, R² et X sont tels que définis précédemment,
le composé de formule (XV) pouvant être soumis à des conditions d'oxydations pour conduire au composé de formule (XX), cas particulier des composés de formule (II): 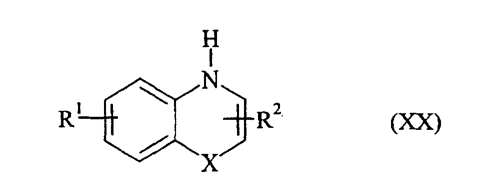 dans laquelle R¹, R² et X sont tels que définis précédemment.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des intermédiaires de synthèse utiles dans la préparation des composés de l'invention.

### EXEMPLE 1: N-[2-(6-Méthoxy-2,3-dihydro-4H-1,4-benzoxazin-4-yl)éthyl] acétamide

### Stade A : 1-Amino-4-méthoxyphénol

Le 4-méthoxy-2-nitrophénol (5 g ; 29,56 mmol) est solubilisé dans le méthanol. Le palladium sur charbon (5 % en masse, 250 mg) est ensuite ajouté. Le mélange est laissé sous agitation, sous 40 psi d'hydrogène, dans l'appareil de Parr pendant 1 heure. La solution est ensuite filtrée sur célite, puis le solvant est éliminé sous pression réduite pour conduire au produit du titre sous la forme d'un solide noir à reflet métallique.
*Point de fusion* : *138-140°C*

### Stade B : N-(2-Hydroxy-5-méthoxyphényl)acétamide

Le composé obtenu au stade A (1g ; 7,19 mmol) est solubilisé dans 16 ml d'eau à l'aide d'acide chlorhydrique concentré (0,58 ml). L'anhydride acétique (1,2 éq ; 8,62 mmol; 813 µl) est ajouté à la solution rouge précédemment obtenue. Le mélange est homogénéisé et versé dans 5 ml d'une solution d'acétate de sodium (1,7 éq ; 12,22 mmol ; 1,075 g). Après 15 minutes d'agitation, le précipité est filtré sur Büchner, puis rincé avec de l'eau. Le solide est ensuite dissous dans l'acétate d'éthyle, puis séché sur MgSO₄. Après filtration, le solvant est éliminé sous pression réduite pour conduire au produit du titre sous la forme d'un solide rouge.
*Point de fusion : 156-157°C*

### Stade C : 1-(6-Méthoxy-2,3-dihydro-4H-1,4-benzoxazin-4-yl)-1-éthanone

En conditions anhydres, le composé obtenu au stade B (1 g ; 5,52 mmol) est solubilisé dans le mélange acétonitrile/dichlorométhane (4/6). L'hydroxyde de sodium (4 éq ; 22,1 mmol ; 883 mg) préalablement broyé, le dibromoéthane (4 éq ; 22,1 mmol ; 1,96 mmol), et l'aliquat 336 (quantité catalytique) sont ajoutés à la solution. On laisse sous agitation, sous argon, à 30°C pendant 24 heures, puis on ajoute 220 mg (1 éq) d'hydroxyde de sodium pour terminer la réaction. La solution est filtrée sur fritté, et le solide brun rincé avec de l'éther. Le filtrat rouge est récupéré, et les solvants éliminés sous pression réduite. Le résidu obtenu est purifié par chromatographie « flash » sur gel de silice (éluant : EP/AcOEt (1/1)). Le produit du titre est obtenu sous forme d'un solide rouge.
*Point de fusion : 76-77°C*

### Stade D : 6-Méthoxy-3,4-dihydro-2H-1,4-benzoxazine

Le composé obtenu au stade C (1,5 g ; 7,26 mmol) est solubilisé dans 7 ml de méthanol, puis de l'eau (14 éq; 101,6 mmol; 1,9 ml) et de l'hydroxyde de potassium (6 éq; 43,5 mmol ; 2,44 g) sont ensuite additionnés. La solution est laissée sous forte agitation, à 60°C pendant 2 heures. Après 3 extractions au dichlorométhane, la phase organique est séchée sur MgSO₄, puis éliminée sous pression réduite. Le résidu obtenu est purifié par chromatographie « flash » sur gel de silice (éluant : EP/AcOEt (1/1)). Le produit du titre est obtenu sous la forme d'un solide rouge.
*Point de fusion : 59-61°C*

### [ou Stade A' : 6-Méthoxy-2H-1,4-benzoxazine 3-one

Le dérivé obtenu au stade A (5,94 g ; 42,7 mmol) est solubilisé dans 60 ml de méthylisobutylcétone, puis une solution d'hydrogénocarbonate de sodium (3 éq; 128,1 mmol; 10,76 g dissous dans 60 ml d'eau) est ensuite ajoutée. Le chlorure de chloroacétyle (1,1 éq ; 46,9 mmol ; 3,74 ml) est alors lentement additionné. Le mélange est maintenu à reflux pendant une heure, puis la méthylisobutylcétone est éliminée sous pression réduite. Le résidu est repris par de l'acétate d'éthyle, et la phase organique est lavée 2 fois à l'eau, séchée sur MgSO₄, puis éliminée sous pression réduite pour conduire au produit du titre sous la forme d'un solide.

### Stade D : 6-Méthoxy-3,4-dihydro-2H-1,4-benzoxazine

Sous atmosphère inerte, le composé obtenu au stade A' (2,1 g; 11,7 mmol) est solubilisé dans 60 ml de tétrahydrofurane, puis l'hydrure mixte de lithium aluminium (5,5 éq; 2,45 g ; 64,5 mmol) est additionné par portion. Le mélange est laissé sous agitation, à température ambiante, pendant 24 heures. La température de la solution est abaissée à 0°C, puis 2,45 ml d'eau, 2,45 ml d'hydroxyde de sodium à 15 % et 7,35 ml d'eau sont lentement additionnés. Le mélange est laissé 30 minutes sous agitation, puis filtré sur Büchner. Après élimination des solvants sous pression réduite, le résidu obtenu est purifié par chromatographie « flash » sur gel de silice (éluant : EP/AcOEt (6/4)). Le produit du titre est obtenu sous forme d'un solide rouge.
*Point de fusion : 59-61 °C*]

### Stade E : 2-(6-Méthoxy-2,3-dihydro-4H-1,4-benzoxazin-4-yl)acétonitrile

Le composé obtenu au stade D (400 mg ; 2,42 mmol) est mis en suspension dans 10 ml d'eau. Le carbonate de potassium (10 éq ; 24,2 mmol ; 3,35 g), le bromure de tétrabutyle ammonium (0,05 éq ; 0,12 mmol ; 40 mg) et le bromoacétonitrile (8 éq ; 19,37 mmol ; 1,35 ml) sont ensuite additionnés. Le mélange est laissé sous forte agitation, à 70°C, pendant 12 heures. Après retour à température ambiante, le produit formé est extrait au dichlorométhane. La phase organique est séchée sur MgSO₄, filtrée, puis concentrée sous pression réduite. Le résidu obtenu est purifié par chromatographie « flash » sur gel de silice (éluant : EP/AcOEt (1/1)). Le produit du titre est obtenu sous forme d'un solide.
*Point de fusion: 73-74°C*

### Stade F : N-[2-(6-Méthoxy-2,3-dihydro-4H-1,4-benzoxazin-4 yl)éthyl]acétamide

Le composé obtenu au stade E (590 mg ; 2,88 mmol) est solubilisée dans le réacteur de Parr avec de l'anhydride acétique. Le nickel de Raney (10 % en masse, 60 mg) et l'acétate de sodium (1,5 éq ; 4,33 mmol ; 355 mg) sont ensuite ajoutés. Le mélange est laissé 12 heures, sous une pression d'hydrogène de 40 psi, à 50°C. Après retour à température ambiante, la solution est filtrée sur célite, et le filtrat évaporé. Le résidu obtenu est repris par l'eau, puis on extrait 3 fois avec de l'acétate d'éthyle. Les phases organiques sont réunies, séchées sur MgSO₄, puis concentrée sous pression réduite. Le brut réactionnel est purifié par chromatographie « flash » sur gel de silice (éluant : EP/AcOEt (4/6) puis AcOEt/MeOH (95/5)). Le produit du titre est obtenu sous forme de cristaux blancs après lavage à l'éther.
*Point de fusion : 107-108°C*

| *Microanalyse élémentaire :* | | | |
|---|---|---|---|
| | *C%* | *H%* | *N%* |
| *Calculé* | *62, 38* | *7, 25* | *11,19* |
| *Trouvé* | *62, 26* | *7,60* | *11,05* |

### EXEMPLE 2 : N-[2-(6-Méthoxy-2,3-dihydro-4H-1,4-benzoxazin-4-yl)éthyl] butanamide

Sous atmosphère inerte, le composé obtenu au stade E de l'Exemple 1 est solubilisé dans l'éther anhydre (1 ml/0,1 mmol) puis l'hydrure double de lithium aluminium (1,5 éq) est additionné par portion. Le mélange est laissé sous agitation, à température ambiante, pendant 3 heures, puis la solution est hydrolysée par de l'eau et de l'hydroxyde de sodium à 15 %. La solution est laissée 30 minutes sous agitation, puis les sels formés sont filtrés sur Büchner. Les solvants sont éliminés sous pression réduite, et l'huile jaune obtenue est placée sous vide dynamique, sous P₂O₅ pendant 2 heures. Sans plus de purification, l'amine obtenue est directement engagée dans la réaction d'acylation.
Sous atmosphère inerte, l'amine est solubilisée dans du dichlorométhane distillé, puis la solution est refroidie à 0°C. La triéthylamine (3 éq), puis le chlorure de butyryle (1,5 éq) sont lentement additionnés. Le mélange est laissé à température ambiante jusqu'à disparition de l'amine, puis la phase organique est lavée avec de l'eau, séchée sur MgSO₄, et les solvants éliminés sous pression réduite. Le résidu obtenu est purifié par chromatographie « flash » sur gel de silice (éluant : EP/AcOEt (2/8)) pour obtenir le produit du titre sous la forme d'un solide qui est recristallisé dans l'éther.
*Point de fusion : 73-74°C*

| *Microanalyse élémentaire :* | | | |
|---|---|---|---|
| | *C %* | *H%* | *N%* |
| *Calculé* | *64,73* | *7,97* | *10,06* |
| *Trouvé* | *64,57* | *8,02* | *10,10* |

### EXEMPLE 3 : N-[2-(6-Méthoxy-2,3-dihydro-4H-1,4-benzoxazin-4-yl)éthyl]-3-butènamide

Sous atmosphère inerte, le composé obtenu au stade E de l'Exemple 1 (294 mg ; 1,44 mmol) est solubilisé dans 14 ml d'éther anhydre, puis l'hydrure double de lithium aluminium (1,5 éq ; 2,16 mmol ; 82 mg) est additionné par portion. Le mélange est laissé sous agitation, à température ambiante, pendant 3 heures, puis la solution est hydrolysée par 82 µl d'eau, 82 µl d'une solution d'hydroxyde de sodium à 15 % et 246 µl d'eau. La solution est laissée 30 minutes sous agitation, puis les sels formés sont filtrés sur Büchner. Les solvants sont éliminés sous pression réduite, et l'huile jaune obtenue est directement engagée dans la réaction suivante.
Sous atmosphère inerte, à -10°C, l'acide vinylacétique (1,5 éq ; 2,16 mmol ; 190 µl) dilué dans 7 ml de dichlorométhane distillé est mis en présence d'EDCI (1,5 éq ; 2,16 mmol 422 mg) et d'HOBt (1,5 éq ; 2,16 mmol ; 292 mg). On laisse sous agitation, sous argon, à -10°C pendant 30 minutes. En parallèle, l'amine est dissoute dans 10 ml de dichlorométhane distillé et mise en présence de triéthylamine (1 éq ; 1,44 mmol ; 200 µl). L'acide vinylacétique activé est ensuite additionné à la solution d'amine libre. Le milieu réactionnel est laissé 1 heure à -10°C, puis 24 heures à température ambiante. Le mélange est ensuite lavé avec une solution d'hydroxyde de sodium IN, de l'eau, séché sur MgSO₄, et le solvant évaporé sous pression réduite. Le résidu obtenu est purifié par chromatographie « flash» (éluant : EP/AcOEt (3/7)). Le produit du titre est obtenu sous forme d'un solide qui est recristallisé dans le mélange Et₂O/*i*-PrOH.
*Point de fusion : 80-81°C*

| *Microanalyse élémentaire :* | | | |
|---|---|---|---|
| | *C%* | *H%* | *N%* |
| *Calculé* | *65,20* | *7,29* | *10,14* |
| *Trouvé* | *64,55* | *7,35* | *9,93* |

### EXEMPLE 4 : N-[2-(6-Méthoxy-2,3-dihydro-4H-1,4-benzoxazin-4-yl)éthyl] cyclopropanecarboxamide

On procède comme dans l'Exemple 2 en remplaçant le chlorure de butyryle par le chlorure de cyclopropanecarbonyle. Recristallisation dans Et₂O/iPrOH (99/1).
*Point de fusion: 121-122°C*

| *Microanalyse élementaire :* | | | |
|---|---|---|---|
| | *C%* | *H%* | *N%* |
| *Calculé* | *65,20* | *7,29* | *10,14* |
| *Trouvé* | *65,04* | *7,36* | *9,88* |

### EXEMPLE 5 : N-12-(6-Méthoxy-2,3-dihydro-4H-1,4-benzoxazin-4-yl)éthyl]-2-furamide

On procède comme dans l'Exemple 2 en remplaçant le chlorure de butyryle par le chlorure de 2-furoyle. Recristallisation dans Et₂O.
*Point de fusion : 95-96°C*

| *Microanalyse élémentaire :* | | | |
|---|---|---|---|
| | *C%* | *H%* | *N%* |
| *Calculé* | *63,57* | *6,00* | *9,27* |
| *Trouvé* | *63,47* | *6,05* | *9,11* |

### EXEMPLE 6 : N-[2-(6-Méthoxy-2,3-dihydro-4H-1,4-benzoxazin-4-yl)éthyl] benzamide

On procède comme dans l'Exemple 2 en remplaçant le chlorure de butyryle par le chlorure de benzoyle. Recristallisation dans Et₂O/iPrOH.
*Point de fusion : 114-117°C*

| *Microanalyse élémentaire :* | | | |
|---|---|---|---|
| | *C%* | *H%* | *N%* |
| *Calculé* | *69,21* | *6,45* | *8,97* |
| *Trouvé* | *68,78* | *6,60* | *8,86* |

### EXEMPLE 7 : N-[2-(6-Hydroxy-2,3-dihydro-4H-1,4-benzoxazin-4-yl)éthyl] acétamide

Sous argon, le composé obtenu dans l'Exemple 1 (342 mg ; 1,37 mmol) est solubilisé dans 8 ml de dichlorométhane distillé. La solution est refroidie à -78°C, puis le tribromure de bore (4 éq ; 5,47 mmol; 5,17 µl) dilué dans 5 ml de dichlorométhane est lentement additionné. Le mélange est laissé 1 heure à -78°C, puis 2 heures à température ambiante. Le milieu réactionnel est hydrolysé par une solution d'hydrogénocarbonate de sodium saturée jusqu'à pH = 7, puis la phase aqueuse est extraite 4 fois avec du dichlorométhane. Les phases organiques sont réunies, puis éliminées sous pression réduite. Le solide obtenu est solubilisé dans le minimum de dichlorométhane, précipité en présence de pentane, filtré puis lavé avec un mélange Et₂O/pentane.
*Point de fusion : 179-181 °C*

| *Microanalyse élémentaire :* | | | |
|---|---|---|---|
| | *C%* | *H%* | *N%* |
| *Calculé* | *61,00* | *6,83* | *11,86* |
| *Trouvé* | *60, 49* | *6,87* | *11,86* |

### EXEMPLE 8 : N-[2-(6-Hydroxy-2,3-dihydro-4H-1,4-benzoxazin-4-yl)éthyl]-2-furamide

Sous argon, le composé obtenu dans l'Exemple 5 (245 mg ; 0,81 mmol) est solubilisé dans 8 ml de dichlorométhane distillé. La solution est refroidie à -78°C, puis le tribromure de bore (4 éq; 3,25 mmol ; 307 µl) dilué dans 5 ml de dichlorométhane est lentement additionné. Le mélange est laissé 1 heure à -78°C, puis 2 heures à température ambiante. Le milieu réactionnel est hydrolysé par une solution de NaHCO₃ saturée jusqu'à pH = 7, puis la phase aqueuse est extraite 4 fois avec du dichlorométhane. Les phases organiques sont réunies, séchées sur MgSO₄, puis concentrées sous pression réduite. Le brut réactionnel est purifié par chromatographie « flash » sur gel de silice (éluant : EP/AcOEt (1/1) puis (1/9)). Le produit du titre est obtenu sous la forme d'un solide après trituration au pentane.
*Point de fusion : 59-62°C*

| *Microanalyse élémentaire :* | | | |
|---|---|---|---|
| | *C%* | *H%* | *N%* |
| *Calculé* | *62,49* | *5,59* | *9,72* |
| *Trouvé* | *63,18* | *5,65* | *9,33* |

### EXEMPLE 9 : N-[2-(6-Ethyl-2,3-dihydro-4H-1,4-benzoxazin-4-yl)éthyl]acétamide

On procède comme dans l'Exemple 1 à partir du 4-éthyl-2-nitrophénol.

### EXEMPLE 10 : N-[2-(6-Méthoxy-2,3-dihydro-4H-1,4-benzothiazin-4-yI)éthyl] acétamide

On procède comme dans l'Exemple 1 à partir du 4-méthoxy-2-nitrobenzènethiol.

### EXEMPLE 11 : N-[2-(6-Méthoxy-2,3-dihydro-4H-1,4-benzothiazin-4-yl)éthyl]-2-phénylacétamide

On procède comme dans l'Exemple 2 à partir du 4-méthoxy-2-nitrobenzènethiol et en remplaçant le chlorure de butyryle par le chlorure de 2-phénylacétyle.

### EXEMPLE 12 : 4-(6-Méthoxy-2,3-dihydro-4H-1,4-benzoxazin-4-yI)-N-méthyl butanamide

### Stade A : 3-(6-Méthoxy-2,3-dihydro-4H-1,4-benzoxazin-4-yl)butanenitrile

On procède comme dans le stade E de l'Exemple 1 en remplaçant le bromoacétonitrile par le 4-bromobutanenitrile.

### Stade B : Acide 4-(6-méthoxy-2,3-dihydro-4H-1,4-benzoxazin-4-yl)butanoïque

Le composé obtenu au stade A est hydrolysé en présence de NaOH.

### Stade C : 4-(6-Méthoxy-2,3-dihydro-4H-1,4-benzoxazin-4-yl)-N-méthylbutanamide

L'acide obtenu au stade B est soumis à l'action de SOCl₂ pour conduire au chlorure d'acide intermédiaire sur lequel on condense la N-méthylamine afin d'obtenir le produit du titre.

### EXEMPLE 13 : N-Cyclobutyl-4-(6-méthoxy-2,3-dihydro-4H-1,4-benzoxazin-4-yl) butanamide

On procède comme dans l'Exemple 12 en remplaçant la N-méthylamine par la *N*-cyclobutylamine.

### EXEMPLE 14 : N-[2-(6-Méthoxy-3-phényl-2,3-dihydro-4H-1,4-benzoxazin-4-yl) éthyl]acétamide

### Stade A : 2-(4-Méthoxy-2-nitrophénoxy)-1-phényl-1-éthanone

Sous atmosphère inerte, le 4-méthoxy-2-nitrophénol (1,5 g ; 8,86 mmol) est solubilisé dans 30 ml d'acétone, puis la 2-bromoacétophénone (1,5 éq ; 13,29 mmol ; 2,79 g) ainsi que le carbonate de potassium (1,2 éq ; 10,64 mmol ; 1,47 g) sont additionnés. Le mélange est laissé sous forte agitation, à reflux, pendant 3 heures. Après retour à température ambiante, le carbonate de potassium est filtré, le filtrat est évaporé et le résidu repris dans un mélange eau/acétate d'éthyle. La phase organique est séchée sur MgSO₄ puis concentrée sous pression réduite. Le brut réactionnel est purifié par chromatographie « flash » sur gel de silice (éluant : EP/AcOEt (8/2)). Le produit du titre est obtenu sous forme d'un solide jaune.
*Point de fusion : 88-90°C*

### Stade B : 6-Méthoxy-3-phényl-3,4-dihydro-2H-1,4-benzoxazine

Le composé obtenu au stade A (3 g ; 10,44 mmol) est solubilisé dans le réacteur de Parr avec un mélange éthanol/tétrahydrofurane (1/1). Le nickel de Raney (30 % en masse, 900 mg) est ensuite additionné. Le mélange est laissé 48 heures, sous une pression d'hydrogène de 50 psi, à 50°C. Après retour à température ambiante, la solution est filtrée sur célite, et le filtrat évaporé. Le brut réactionnel est purifié par chromatographie « flash » sur gel de silice (éluant : EP/AcOEt (85/15)). Le produit du titre est obtenu sous forme d'un solide jaune.
*Point de fusion : 79-81°C*

### Stade C : 2-(6-Méthoxy-3-phényl-2,3-dihydro-4H-1,4-benzoxazin-4-yl)acétonitrile

Le composé obtenu au stade B (480 mg ; 1,99 mmol) est mis en suspension dans 8 ml d'eau (4 ml/1 mmol). L'hydrogénocarbonate de potassium (10 éq ; 19,89 mmol ; 1,67 g), le bromure de tétrabutylammonium (1 éq ; 1,99 mmol ; 641 mg) et le bromoacétonitrile (8 éq ; 15,91 mmol ; 1,11 ml) sont ensuite additionnés. Le mélange est laissé sous forte agitation, à 90°C, pendant 8 heures. Après retour à température ambiante, le produit formé est extrait au dichlorométhane. La phase organique est séchée sur MgSO₄, filtrée, puis concentrée sous pression réduite. Le résidu obtenu est purifié par chromatographie « flash » sur gel de silice (éluant : EP/AcOEt (8/2)). Le produit du titre est obtenu sous la forme d'une huile incolore.

### Stade D : N-[2-(6-Méthoxy-3-phényl-2,3-dihydro-4H-1,4-benzoxazin-4-yl) éthyl]acétamide

Le composé obtenu au stade C (200 mg ; 0,71 mmol) est solubilisé dans le réacteur de Parr avec de l'anhydride acétique. Le nickel de Raney (30 % en masse, 60 mg) et l'acétate de sodium (1,5 éq ; 1,07 mmol ; 88 mg) sont ensuite ajoutés. Le mélange est laissé 36 heures, sous une pression d'hydrogène de 40 psi, à 50°C. Après retour à température ambiante, la solution est filtrée sur célite, et le filtrat évaporé. Le brut réactionnel est purifié par chromatographie « flash » sur gel de silice (éluant : AcOEt). Le produit du titre est obtenu sous forme de cristaux blancs qui sont recristallisés dans le mélange Et₂O/*i*-PrOH.
*Point de fusion : 125-126°C*

| *Microanalyse élémentaire :* | | | |
|---|---|---|---|
| | *C %* | *H%* | *N%* |
| *Calculé* | *69,92* | *6, 79* | *8,58* |
| *Trouvé* | *70, 00* | *6,95* | *8,50* |

### EXEMPLE 15 : N-[2-(6-Méthoxy-3-phényl-2,3-dihydro-4H-1,4-benzoxazin-4-yl) éthyl]benzamide

Sous atmosphère inerte, le composé obtenu au stade C de l'Exemple 14 est solubilisé dans l'éther anhydre (1 ml/0,1 mmol), puis l'hydrure mixte de lithium aluminium (1,5 éq) est additionné par portion. Le mélange est laissé sous agitation, à température ambiante, pendant 3 heures, puis la solution est hydrolysée, à 0°C, par de l'eau et une solution d'hydroxyde de sodium à 15 %. La solution est laissée 30 minutes sous agitation, puis les sels formés sont filtrés sur Büchner. Les solvants sont éliminés sous pression réduite, et l'huile jaune obtenue est placée sous vide dynamique, sous P₂O₅ pendant 2 heures. Sans plus de purification, l'amine obtenue est directement engagée dans la réaction d'acylation.
Sous atmosphère inerte, l'amine est solubilisée dans du dichlorométhane distillé, puis la solution est refroidie à 0°C. La triéthylamine (3 éq), puis le chlorure de benzoyle (1,5 éq) sont lentement additionnés. Le mélange est laissé à température ambiante jusqu'à disparition de l'amine, puis la phase organique est lavée avec de l'eau, séchée sur MgSO₄, et les solvants éliminés sous pression réduite. Le résidu obtenu est purifié par chromatographie « flash » sur gel de silice (éluant EP/AcOEt (7,3) puis (6/4)) pour obtenir une huile donnant un solide après cristallisation dans du CH₂Cl₂.
*Point de fusion : 61-62°C*

| *Microanalyse élémentaire :* | | | |
|---|---|---|---|
| | *C %* | *H%* | *N%* |
| *Calculé* | *74,21* | *6,23* | *7,21* |
| *Trouvé* | *73,86* | *6,25* | *7,11* |

### EXEMPLE 16 : N-[2-(6-Méthoxy-3-phényl-2,3-dihydro-4H-1,4-benzoxazin-4-yI) éthyl]-2-furamide

On procède comme dans l'Exemple 15 en remplaçant le chlorure de benzoyle par le chlorure de 2-furoyle.
*Point de fusion : 52-54°C*

| *Microanalyse élémentaire :* | | | |
|---|---|---|---|
| | *C%* | *H%* | *N%* |
| *Calculé* | *69,83* | *5,86* | *7,40* |
| *Trouvé* | *69,62* | *5,90* | *7,14* |

### EXEMPLE 17 : N-[2-(6-Méthoxy-3-phényl-2,3-dihydro-4H-1,4-benzoxazin-4-yl) éthyl]butanamide

On procède comme dans l'Exemple 15 en remplaçant le chlorure de benzoyle par le chlorure de butyryle.
*Point de fusion : 60-62°C*

| *Microanalyse élémentaire* : | | | |
|---|---|---|---|
| | *C%* | *H%* | *N%* |
| *Calculé* | *71,16* | *7,39* | *7,90* |
| *Trouvé* | *70,68* | *7,33* | *7,77* |

### EXEMPLE 18 : N-(2-{6-Méthoxy-3-[3-(trifluorométhyl)phényl]-2,3-dihydro-4H-1,4-benzoxazin-4-yl}éthyl)cyclopropanecarboxamide

### Stade A : 2-{6-Méthoxy-3-[3-(trifluorométhyl)phényl]-2,3-dihydro-4H-1,4-benzoxazin-4-yl}acétonitrile

On procède comme dans les stades A, B et C de l'Exemple 14 en remplaçant au stade A la 2-bromoacétophénone par la 2-bromo-1-[3-(trifluorométhyl)phényl]-1-éthanone.

### Stade B : N-(2-{6-Méthoxy-3-[3-(trifluorométhyl)phényl]-2,3-dihydro-4H-1,4-benzoxazin-4-yl}éthyl)cyclopropanecarboxamide

On procède comme dans l'Exemple 15 à partir du composé obtenu au stade A et en remplaçant le chlorure de benzoyle par le chlorure de cyclopropylcarbonyle.

### EXEMPLE 19 : N-[-2-(3-Benzyl-6-méthoxy-2,3-dihydro-4H-1,4-benzoxazin-4-yI) éthyl]acétamide

On procède comme dans l'Exemple 14 en remplaçant au stade A la 2-bromoacétophénone par la 1-bromo-3-phénylacétone.

### EXEMPLE 20 : 2-Méthyl-N-[2-(3-phényl-2,3-dihydro-4H-1,4-benzothiazin-4-yl) éthyl]propanamide

### Stade A : 2-(3-Phényl-2,3-dihydro-4H-1,4-benzothiazin-4-yl)acétonitrile

On procède comme dans les stades A, B et C de l'Exemple 14 à partir du 4-méthoxy-2-nitrobenzènethiol.

### Stade B : 2-Méthyl-N-[2-(3-phényl-2,3-dihydro-4H-1,4-benzothiazin-4-yl) éthyl]propanamide

On procède comme dans l'Exemple 15 à partir du composé obtenu au stade A et en remplaçant le chlorure de benzoyle par le chlorure d'isobutyryle.

### EXEMPLE 21 : 4-(6-Méthoxy-3-phényl-2,3-dihydro-4H-1,4-benzoxazin-4-yI) -N-méthylbutanamide

### Stade A : 4-(6-Méthoxy-3-phényl-2,3-dihydro-4H-1,4-benzoxazin-4-yl)butanenitrile

On procède comme dans les stades A, B et C de l'Exemple 14 en remplaçant au stade C le bromoacétonitrile par le 4-bromobutanenitrile.

### Stade B : 4-(6-Méthoxy-3-phényl-2,3-dihydro-4H-1,4-benzoxazin-4-yl)-N-méthyl butanamide

On procède comme dans les stades B et C de l'Exemple 12.

### EXEMPLE 22 : N-Hexyl-4-(6-méthoxy-3-phényl-2,3-dihydro-4H-1,4-benzothiazin-4- yl)butanamide

On procède comme dans l'Exemple 21 en remplaçant la *N*-méthylamine par la *N*-hexylamine.

### EXEMPLE 23 : N-[2-(6-Méthoxy-2-phényl-2,3-dihydro-4H-1,4-benzoxazin-4-yl) éthyl]acétamide

### Stade A : 2-Bromo-N-(2-hydroxy-5-méthoxyphényl)-2-phénylacétamide

A une solution du composé obtenu au stade A de l'Exemple 1 (3,77 g ; 27,13 mmol), solubilisée dans 33 ml d'acétate d'éthyle (1,2 ml/1 mmol), en présence de 33 ml d'eau (1,2 ml/1 mmol) et d'hydrogénocarbonate de sodium (1,5 éq; 40,7 mmol ; 3,42 g), est additionné lentement le chlorure de 2-bromophénylacétyle (1,2 éq ; 32,55 mmol ; 7,60 g), à 0°C. Le mélange est laissé 2 heures sous agitation. Après ajout d'acétate d'éthyle au milieu réactionnel, la phase organique est lavée une fois avec de l'eau, séchée sur MgSO₄, et concentrée sous pression réduite. Le brut réactionnel est purifié par chromatographie « flash » sur gel de silice après dépôt solide (éluant : EP/AcOEt (7/3) puis (6/4)). Le produit du titre est obtenu sous forme d'un solide marron qui est recristallisé dans le mélange Et₂O/i-PrOH.
*Point de fusion* : 145-147°C

### Stade B : 6-Méthoxy-2-phényl-2H-1,4-benzoxazin-3(4H)-one

Le composé obtenu au stade A (5 g; 14,87 mmol) est solubilisé dans 15 ml de diméthylformamide (1 ml/1 mmol), puis du carbonate de potassium (1,5 éq ; 22,31 mmol ; 3,08 g) est additionné. La solution est laissée sous agitation, à température ambiante, pendant 2,5 heures, puis 60 ml d'eau sont ajoutés et un précipité est formé. Après une nuit au réfrigérateur, le solide est filtré sur fritté, rincé à l'eau, séché sous vide dynamique, sous P₂O₅ pour conduire au produit du titre qui est engagé dans la réaction suivante sans plus de purification.

### Stade C : 6-Méthoxy-2-phényl-3,4-dihydro-2H-1,4-benzoxazine

Sous atmosphère inerte, le composé obtenu au stade B (3,26 g ; 12,77 mmol) est solubilisé dans 70 ml de tétrahydrofurane anhydre (6 ml/1 mmol), puis l'hydrure mixte de lithium aluminium (5 éq ; 63,85 mmol ; 2,42 g) est additionné par portion, à 0°C. Le mélange est laissé sous agitation, à température ambiante, pendant 16 heures, puis la solution est hydrolysée à 0°C par 2,4 ml d'eau, 2,4 ml d'une solution d'hydroxyde de sodium à 15 %, et 7,2 ml d'eau. La solution est laissée 30 minutes sous agitation, puis les sels formés sont filtrés sur Büchner, et le solvant éliminé sous pression réduite. Le brut réactionnel est purifié par chromatographie « flash » sur gel de silice (éluant : EP/AcOEt (8/2)). Le produit du titre est obtenu sous forme d'un solide.
*Point de fusion : 95-100°C*

### Stade D : 2-(6-Méthoxy-2-phényl-2,3-dihydro-4H-1,4-benzoxazin-4-yl)acétonitrile

Le composé obtenu au stade C (1,5 g ; 6,22 mmol) est mis en suspension dans 25 ml d'eau (4 ml/1 mmol). L'hydrogénocarbonate de potassium (10 éq ; 62,2 mmol ; 5,22 g), le bromure de tétrabutylammonium (0,05 éq ; 0,31 mmol ; 100 mg) et le bromoacétonitrile (8 éq ; 49,7 mmol ; 3,46 ml) sont ensuite additionnés. Le mélange est laissé sous forte agitation, à 70°C, pendant 16 heures. Après retour à température ambiante, le produit formé est extrait au dichlorométhane. La phase organique est séchée sur MgSO₄, filtrée, puis concentrée sous pression réduite. Le résidu obtenu est purifié par chromatographie « flash » sur gel de silice (éluant : EP/AcOEt (8/2)). Le produit du titre est obtenu sous forme d'un solide.
*Point de fusion : 154-156°C*

### Stade E : N-[2-(6-Méthoxy-2-phényl-2,3-dihydro-4H-1,4-benzoxazin-4-yl) éthyl]acétamide

Le composé obtenu au stade D (350 mg ; 1,25 mmol) est solubilisé dans le réacteur de Parr avec de l'anhydride acétique. Le nickel de Raney (30 % en masse, 115 mg) et l'acétate de sodium (1,5 éq ; 1,87 mmol; 154 mg) sont ensuite ajoutés. Le mélange est laissé 16 heures, sous une pression d'hydrogène de 40 psi, à 50 °C. Après retour à température ambiante, la solution est filtrée sur célite, et le filtrat évaporé. Le brut réactionnel est purifié par chromatographie « flash » sur gel de silice (éluant: EP/AcOEt (1/9)). Le produit du titre est obtenu sous forme de cristaux blancs et recristallisé dans le mélange Et₂O/*i*-PrOH (6/4).
*Point de fusion: 153-154°C*

| *Microanalyse élémentaire :* | | | |
|---|---|---|---|
| | *C%* | *H%* | *N %* |
| *Calculé* | *69,92* | *6,79* | *8,58* |
| *Trouvé* | *69,94* | *6,75* | *8,59* |

### EXEMPLE 24 : N-[2-(6-Méthoxy-2-phényl-2,3-dihydro-4H-1,4-benzoxazin-4-yl) éthyl]benzamide

Sous atmosphère inerte, le composé obtenu au stade D de l'Exemple 23 est solubilisé dans l'éther anhydre (2 ml/0,1 mmol) puis l'hydrure mixte de lithium aluminium (1,5 éq) est additionné par portion. Le mélange est laissé sous agitation, à température ambiante, pendant 2 heures, puis la solution est hydrolysée, à 0°C, par de l'eau et une solution d'hydroxyde de sodium à 15 %. La solution est laissée 30 minutes sous agitation, puis les sels formés sont filtrés sur Büchner. Les solvants sont éliminés sous pression réduite, et l'huile jaune obtenue est placée sous vide dynamique, sous P₂O₅ pendant 2 heures. Sans plus de purification, l'amine obtenue est directement engagée dans la réaction d'acylation.
Sous atmosphère inerte, l'amine est solubilisée dans du dichlorométhane distillé, puis la solution est refroidie à 0°C. La triéthylamine (3 éq), puis le chlorure de benzoyle (1,5 éq) sont lentement additionnés. Le mélange est laissé à température ambiante jusqu'à disparition de l'amine, puis la phase organique est lavée avec de l'eau, séchée sur MgSO₄, et les solvants éliminés sous pression réduite. Le résidu obtenu est purifié par chromatographie « flash » sur gel de silice pour obtenir un solide qui est recristallisé dans Et₂O/AcOEt (99/1).
*Point de fusion : 174-175°C*

| *Microanalyse élémentaire :* | | | |
|---|---|---|---|
| | *C%* | *H%* | *N%* |
| *Calculé* | *74,21* | *6,23* | *7,21* |
| *Trouvé* | *73,56* | *6,26* | *7,01* |

### EXEMPLE 25 : N-[2-(6-Méthoxy-2-phényl-2,3-dihydro-4H-1,4-benzoxazin-4-yl) éthyl]butanamide

On procède comme dans l'Exemple 24 en remplaçant le chlorure de benzoyle par le chlorure de butyryle. Recristallisation dans Et₂O/iPrOH.
*Point de fusion : 124-125°C*

| *Microanalyse élémentaire :* | | | |
|---|---|---|---|
| | *C %* | *H%* | *N%* |
| *Calculé* | *71,16* | *7,39* | *7,90* |
| *Trouvé* | *70,59* | *7,39* | *7,70* |

### EXEMPLE 26 : N-[2-(6-Méthoxy-2-phényl-2,3-dihydro-4H-1,4-benzoxazin-4-yl) éthyl]-2-furamide

On procède comme dans l'Exemple 24 en remplaçant le chlorure de benzoyle par le chlorure de 2-furoyle. Recristallisation dans Et₂O/AcOEt (99/1).
*Point de fusion : 129-130°C*

| *Microanalyse élémentaire :* | | | |
|---|---|---|---|
| | *C%* | *H%* | *N%* |
| *Calculé* | *69,83* | *5,86* | *7,40* |
| *Trouvé* | *69,22* | *5,99* | *7,12* |

### EXEMPLE 27 : N-[2-(6-Méthoxy-2-phényl-2,3-dihydro-4H-1,4-benzoxazin-4-yl) éthyl]cyclopropanecarboxamide

On procède comme dans l'Exemple 24 en remplaçant le chlorure de benzoyle par le chlorure de cyclopropanecarbonyle.

### EXEMPLE 28 : N-[2-(6-Methoxy-2-phényl-2,3-dihydro-4H-1,4-benzoxazin4-yl) éthyl]-N'-phénylurée

A une suspension de 0,01 mole du chlorhydrate de l'amine obtenue dans l'Exemple 24 (avant acylation) dans 5 cm³ de pyridine sont ajoutées 0,011 moles d'isocyanate de phényle. Après 1 heure d'agitation à 80°C, le milieu réactionnel est versé sur de l'eau glacée, puis acidifié par HCI 1N. Le précipité formé est essoré, lavé, séché puis recristallisé pour conduire au produit du ditre.

### EXEMPLE 29 : N-[2-(2-Acétyl-6-méthoxy-2,3-dihydro-4H-1,4-benzoxazin-4-yl) éthyl]pentanamide

### Stade A : 2-(2-Acétyl-6-méthoxy-2,3-dihydro-4H-1,4-benzoxazin-4-yl) acétonitrile

On procède comme dans l'Exemple 23 en remplaçant au stade A le chlorure de 2-bromophénylacétyle par le chlorure de 2-bromo-3-oxobutanoyle.

### Stade B : N-[2-(2-Acétyl-6-méthoxy-2,3-dihydro-4H-1,4-benzoxazin-4-yl)éthyl] pentanamide

On procède comme dans l'Exemple 24 en remplaçant le chlorure de benzoyle par le chlorure de pentanoyle.

### EXEMPLE 30 : N-(2-Furylméthyl)4-(6-méthoxy-2-phényl-2,3-dihydro-4H-1,4-benzoxazin-4-yl)butanamide

### Stade A : 2-(6-Méthoxy-2-phényl-2,3-dihydro-4H-1,4-benzoxazin-4-yl) butanenitrile

On procède comme dans les stades A, B, C et D de l'Exemple 23 en remplaçant, au stade D le bromoacétonitrile par le 4-bromobutanenitrile.

### Stade B : N-(2-Furylméthyl)-4-(6-méthoxy-2-phényl-2,3-dihydro-4H-1,4-benzoxazin-4-yl)butanamide

On procède comme dans les stades B et C de l'Exemple 12 en remplaçant au stade C la N-méthylamine par la N-(2-furylméthyl)amine.

### EXEMPLE 31 : N-[2-(6-Cyclopropyl-2,3-dihydro-4H-1,4-benzoxazin-4-yl)éthyl] cyclohexanecarboxamide

### Stade A : 2-(6-Cyclopropyl-2,3-dihydro-4H-1,4-benzoxazin-4-yl)acétonitrile

On procède comme dans l'Exemple 1 (stades A-E) en partant du 4-cyclopropyl-2-nitrophénol.

### Stade B : N-[2-(6-Cyclopropyl-2,3-dihydro-4H-1,4-benzoxazin-4-yl)éthyl] cyclohexanecarboxamide

On procède comme dans l'Exemple 2 à partir du composé obtenu au stade A, et en remplaçant le chlorure de butyryle par le chlorure de cyclohexylcarbonyle.

### EXEMPLE 32 : N-[2-(6-Méthoxy-4H-1,4-benzoxazin-4-yl)éthyl]acétamide

On procède comme dans l'Exemple 1, le composé obtenu au stade D étant soumis, après protection de la fonction NH par un groupement Boc ((*tert*-butyl)oxycarbonyl), à l'action successive de NBS en présence d'AIBN, puis de NaI dans l'acétone, pour conduire, après déprotection à l'acide formique, au 6-méthoxy-4*H*-1,4-benzoxazine.
On poursuit ensuite comme dans les stades E et F de l'Exemple 1.

### EXEMPLE 33 : N-(2-{6-[(Méthylamino)sulfonyl]-4H-1,4-benzoxazin-4-yl}éthyl) acétamide

On procède comme dans l'Exemple 32 en utilisant comme produit de départ le 4-hydroxy-*N*-méthyl-3-nitrobenzènesulfonamide.

### EXEMPLE 34 : N-[2-(6-Méthoxy-4H-1,4-benzoxazin-4-yl)éthyl]cyclopropanecarboxamide

On procède comme dans l'Exemple 32 en utilisant au stade F le procédé utilisé dans l'Exemple 2 en remplaçant le chlorure de butyryle par le chlorure de cyclopropanecarbonyle.

### EXEMPLE 35 : 4-{2-[(Cyclopropylcarbonyl)amino]éthyl}-4H-1,4-benzoxazin-6-yl carbamate de méthyle

On procède comme dans l'Exemple 34 en prenant comme produit de départ le 4-hydroxy-3-nitrophénylcarbamate de méthyle.

### EXEMPLE 36 : N-{2-[2-(3-Aminophényl)-6-méthoxy-4H-1,4-benzoxazin-4-yl]éthyl} acétamide

On procède comme dans l'Exemple 23 en remplaçant au stade A le chlorure de bromo(phényl)acétyle par le chlorure de (3-aminophényl)(bromo)acétyle et en soumettant le composé obtenu au stade C à l'action successive de NBS/AIBN et NaI avant de poursuivre les stades D et E.

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la *pars tuberalis* de mouton. La *pars tuberalis* de l'adénohypophyse est en effet caractérisée, chez les mammifères, par une haute densité en récepteurs de la mélatonine (Journal of Neuroendocrinology, 1, pp. 1-4, 1989).

### Protocole

1) Les membranes de *pars tuberalis* de mouton sont préparées et utilisées comme tissu cible dans des expériences de saturation pour déterminer les capacités et affinités de liaison pour la 2-[¹²⁵I]-iodomélatonine.
2) Les membranes de *pars tuberalis* de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : 1. Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

### 2. Etude de liaison aux sites de liaisons MT₃ de la melatonine

Les expériences de liaison sur les sites *MT*_{*3*} sont réalisées sur membranes de cerveau de hamster en utilisant la 2-[¹²⁵I] iodomélatonine comme radioligand. Les membranes sont incubées pendant 30 minutes avec la 2-[¹²⁵I] iodomélatonine à la température de 4°C et différentes concentrations des composés à tester. Après l'incubation, les membranes sont rapidement filtrées puis lavées par du tampon froid à l'aide d'un système de filtration. La radioactivité fixée est mesurée par un compteur à scintillation. Les valeurs d'IC₅₀ (concentration inhibant de 50 % la liaison spécifique) sont calculées à partir des courbes de compétition selon un modèle de régression non linéaire.

Ainsi, les valeurs d'IC₅₀ trouvées pour les composés de l'invention attestent d'une liaison pour l'un ou l'autre des sites de liaisons mélatoninergiques, ces valeurs étant ≤ 10 µM.

### EXEMPLE D: Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12: 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de *N*-[2-(6-méthoxy-2,3-dihydro-4*H*-1,4-benzoxazin -4-yl)éthyl]benzamide (Exemple 6) | 5 g |
| Amidon de blé | 20 g |
| Amidon de maïs | 20 g |
| Lactose | 30g |
| Stéarate de magnésium | 2 g |
| Silice | 1 g |
| Hydroxypropylcellulose | 2 g |

## Revendications

1. Composés de formule (I): 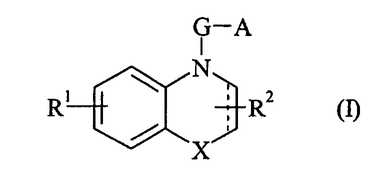 dans laquelle :
◆ R¹ représente un atome d'halogène ou un groupement R, OR, SR, SO₂NRR', -NRR', 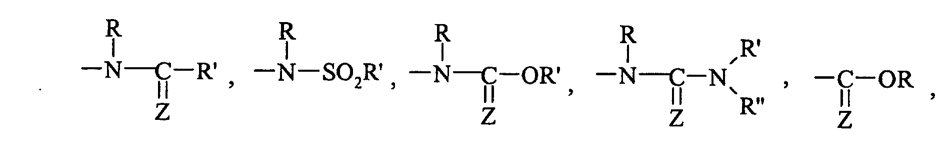 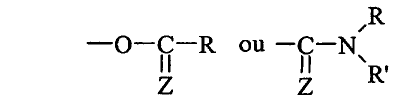 (où Z représente un atome de soufre ou d'oxygène et R, R' et R", identiques ou différents, représentent un atome d'hydrogène ou un groupement alkyle (C₁-C₆) linéaire ou ramifié substitué ou non substitué, alkényle (C₂-C₆) linéaire ou ramifié substitué ou non substitué, alkynyle (C₂-C₆) linéaire ou ramifié substitué ou non substitué, cycloalkyle (C₃-C₈) substitué ou non substitué, cycloalkyle (C₃-C₈) alkyle (C₁-C₆) linéaire ou ramifié substitué ou non substitué, aryle, arylalkyle (C₁-C₆) linéaire ou ramifié, hétéroaryle ou hétéroarylalkyle (C₁-C₆) linéaire ou ramifié,
(R et R') ou (R' et R") pouvant former ensemble, avec l'atome d'azote qui les porte un groupement morpholinyle, pipéridinyle, pipérazinyle ou pirrolidinyle),
◆ G représente un groupement (CH₂)ₙ dans lequel n vaut 2 ou 3
◆ A représente un groupement 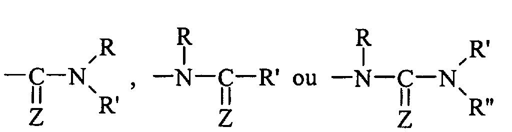 (où R, R', R" et Z sont tels que définis précédemment),
◆ R² représente un atome d'halogène ou un groupement R, OR, COR, COOR ou OCOR (où R est tel que défini précédemment),
◆ X représente un atome d'oxygène ou de soufre,
◆ la notation ---- signifie que la liaison est simple ou double, étant entendu que la valence des atomes est respectée,
étant entendu que :
- sauf précision contraire, les groupements comportant une partie alkyle dans leur structure contiennent de 1 à 6 atomes de carbone et peuvent être linéaires ou ramifiés,
- le terme « substitué » affecté aux expression « alkyle », « alkényle », « alkynyle », « cycloalkyle » signifie que ces groupements peuvent être substitués par un ou plusieurs groupements, identiques ou différents, choisis parmi hydroxy, alkoxy, alkyle, polyhalogénoalkyle, amino (non substitué ou substitué par un ou deux groupements alkyle (C₁-C₆) linéaire ou ramifié) et atomes d'halogène,
- le terme « substitué » affecté à l'expression « cycloalkylalkyle » signifie que la partie cyclique du groupement est substituée par un ou plusieurs groupements, identiques ou différents, choisis parmi hydroxy, alkoxy, alkyle, polyhalogénoalkyle, amino (non substitué ou substitué par un ou deux groupements alkyle (C₁-C₆) linéaire ou ramifié) et atomes d'halogène,
- par « aryle » on entend un groupement phényle, naphtyle ou biphényle, ces groupements étant non substitués ou substitués par un ou plusieurs groupements, identiques ou différents, choisis parmi hydroxy, alkoxy, alkyle, amino, alkylamino, dialkylamino, nitro, cyano, polyhalogénoalkyle, formyle, carboxy, alkyloxycarbonyle, amido, et atomes d'halogène,
- par « hétéroaryle » on entend tout groupement aromatique mono ou bi-cyclique, contenant un à trois hétéroatomes choisis parmi oxygène, soufre et azote, ces groupements étant non substitués ou substitués par un ou plusieurs groupements, identiques ou différents, choisis parmi hydroxy, alkoxy, alkyle, amino, alkylamino, dialkylamino, nitro, cyano, polyhalogénoalkyle, formyle, carboxy, alkyloxycarbonyle, amido, et atomes d'halogène,
leurs énantiomères et diastéréoisomères, ainsi que leurs sels d'addition à un acide ou à une base pharmaceutiquement acceptable.

2. Composés de formule (I) selon la revendication 1 pour lesquels X représente un atome d'oxygène, leurs énantiomères et diastéréoisomères, ainsi que leurs sels d'addition à un acide ou à une base pharmaceutiquement acceptable.

3. Composés de formule (I) selon la revendication 1 qui sont des dérivés de (dihydro)benzoxazines, leurs énantiomères et diastéréoisomères, ainsi que leurs sels d'addition à un acide ou à une base pharmaceutiquement acceptable.

4. Composés de formule (I) selon la revendication 1 pour lesquels R¹ représente un groupement OR, leurs énantiomères et diastéréoisomères, ainsi que leurs sels d'addition à un acide ou à une base pharmaceutiquement acceptable.

5. Composés de formule (I) selon la revendication 1 pour lesquels R² représente un atome d'hydrogène, leurs énantiomères et diastéréoisomères, ainsi que leurs sels d'addition à un acide ou à une base pharmaceutiquement acceptable.

6. Composés de formule (I) selon la revendication 1 pour lesquels R² représente un groupement phényle substitué ou non, leurs énantiomères et diastéréoisomères, ainsi que leurs sels d'addition à un acide ou à une base pharmaceutiquement acceptable.

7. Composés de formule (I) selon la revendication 1 pour lesquels A représente un groupement NHCOR, leurs énantiomères et diastéréoisomères, ainsi que leurs sels d'addition à un acide ou à une base pharmaceutiquement acceptable.

8. Composés de formule (I) selon la revendication 1 pour lesquels A représente un groupement CONHR, leurs énantiomères et diastéréoisomères, ainsi que leurs sels d'addition à un acide ou à une base pharmaceutiquement acceptable.

9. Composés de formule (I) selon la revendication 1 qui sont le *N*-[2-(6-hydroxy-2,3-dihydro-4*H*-1,4-benzoxazin-4-yl)éthyl]acétamide, le *N*-[2-(6-méthoxy-2,3-dihydro-4*H*-1,4-benzoxazin-4-yl)éthyl]acétamide, le *N*-[2-(6-méthoxy-2,3-dihydro-4*H*-1,4-benzoxazin-4-yl)éthyl]butanamide, le *N-*[2-(6-méthoxy-2,3-dihydro-4*H*-1,4-benzoxazin-4-yl)éthyl] -3-buténamide, le *N*-[2-(6-méthoxy-2,3-dihydro-4*H*-1,4-benzoxazin-4-yl)éthyl]cyclopropanecarboxamide, le *N*-[2-(6-méthoxy-2,3-dihydro-4*H*-1,4-benzoxazin-4-yl)éthyl]-2-furamide, le *N*-[2-(6-méthoxy-2,3-dihydro-4*H*-1,4-benzoxazin-4-yl)éthyl]benzamide, le *N*-[2-(6-hydroxy-2,3-dihydro-4*H*-1,4-benzoxazin-4-yl)éthyl]-2-furamide, leurs énantiomères et diastéréoisomères, ainsi que leurs sels d'addition à un acide ou à une base pharmaceutiquement acceptable.

10. Composés de formule (I) selon la revendication 1 qui sont le *N*-[2-(6-méthoxy-3-phényl-2,3-dihydro-4*H*-1,4-benzoxazin-4-yl)éthyl]acétamide,le *N*-[2-(6-méthoxy-3-phényl-2,3-dihydro-4*H*-1,4-benzoxazin-4-yl)éthyl]benzamide,le *N*-[2-(6-méthoxy-3-phényl-2,3-dihydro-4*H*-1,4-benzoxazin-4-yl)éthyl]-2-furamide,le *N*-[2-(6-méthoxy-3-phényl-2,3-dihydro-4*H*-1,4-benzoxazin-4-yl)éthyl]butanamide, le *N*-[2-(6-méthoxy-2-phényl-2,3-dihydro-4*H*-1,4-benzoxazin-4-yl)éthyl]acétamide, le *N*-[2-(6-méthoxy-2-phényl-2,3-dihydro-4*H*-1,4-benzoxazin-4-yl)éthyl]-2-furamide, le *N*-[2-(6-méthoxy-2-phényl-2,3-dihydro-4*H*-1,4-benzoxazin-4-yl)éthyl]benzamide, le *N*-[2-(6-méthoxy-2-phényl-2,3-dihydro-4*H*-1,4-benzoxazin-4-yl)éthyl]butanamide,le *N*-[2-(6-méthoxy-2-phényl-2,3-dihydro-4*H*-1,4-benzoxazin-4-yl)éthyl]cyclopropanecarboxamide, leurs énantiomères et diastéréoisomères, ainsi que leurs sels d'addition à un acide ou à une base pharmaceutiquement acceptable.

11. Procédé de préparation des composés de formule (I) selon la revendication 1 **caractérisé en ce que** l'on utilise comme produit de départ le composé de formule (II): 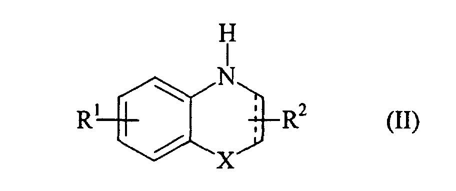 dans laquelle R¹, R², X et la représentation ---- sont tels que définis dans la revendication 1,
sur lequel on fait réagir, en milieu basique, le composé de formule (III):
Br-G-CN (III)
dans laquelle G est tel que défini dans la revendication 1,
pour conduire au composé de formule (IV): 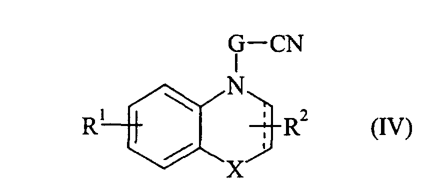 dans laquelle R¹, R², X, G et la représentation ---- sont tels que définis précédemment,
que l'on soumet à une hydrolyse, dans des conditions acides ou basiques afin de conduire au composé de formule (V): 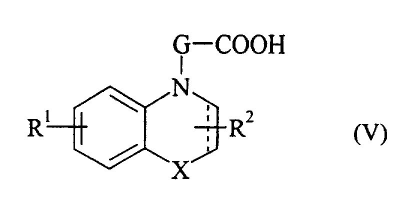 dans laquelle R¹, R², X, G et la représentation ---- ont la même définition que précédemment,
sur lequel on fait agir, en présence d'un agent de couplage ou après transformation en chlorure d'acide correspondant, une amine HNRR' où R et R' sont tels que définis dans la revendication 1, pour conduire au composé de formule (I/a), cas particulier des composés de formule (I): 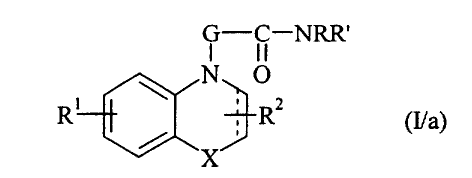 dans laquelle R¹, R², X, G, R, R' et la représentation ---- sont tels que définis précédemment,
composé de formule (I/a) qui est soumis, dans le cas où R et R' représentent simultanément un atome d'hydrogène, à l'action de NaOBr afin de conduire, après hydrolyse, au composé de formule (VI): 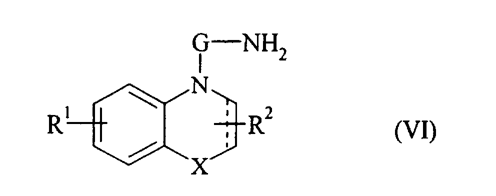 dans laquelle R¹, R², X, G et la représentation ---- sont définis comme précédemment,
composés de formule (VI) (pouvant par ailleurs être obtenus par réduction du composé de formule (IV)) que l'on soumet à l'action :
- d'un chlorure d'acyle de formule (VII) : 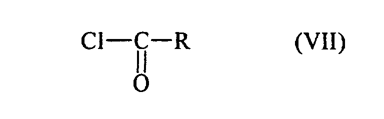 où R est tel que défini précédemment, ou l'anhydride d'acide (mixte ou symétrique) correspondant,
pour obtenir le composé de formule (I/b), cas particulier des composés de formule (I): 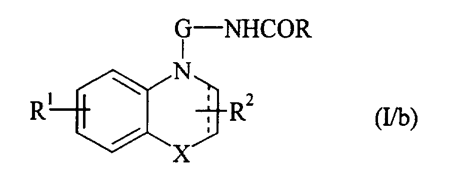 dans laquelle R¹, R², R, X, G et la représentation ---- ont la même définition que précédemment,
- ou d'un composé de formule (VIII):
O=C=N-R (VIII)
dans laquelle R est tel que défini précédemment,
pour conduire au composé de formule (I/c), cas particulier des composés de formule (I): 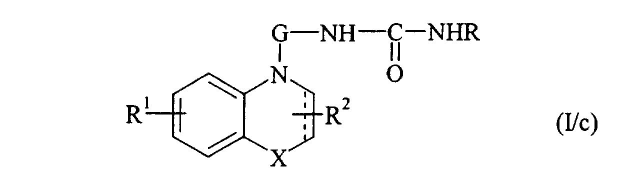 dans laquelle R¹, R², R, X, G et la représentation ---- sont définis comme précédemment,
les composés de formules (I/b) et (I/c) pouvant être soumis à l'action d'un composé de formule (IX):
Rₐ - J (IX)
dans laquelle Rₐ peut prendre toutes les valeurs de R à l'exception de l'atome d'hydrogène et J représente un groupe partant comme un atome d'halogène ou un groupement tosyle,
pour conduire au composé de formule (I/d), cas particulier des composés de formule (I): 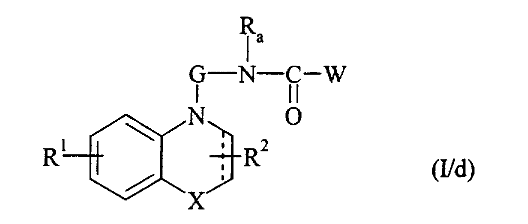 dans laquelle R¹, R², X, G, Rₐ et la représentation ---- ont la même définition que précédemment et W représente un groupement R ou -NRR' où R et R' sont tels que définis précédemment,
et/ou les composés de formules (I/a), (I/b), (I/c) et (I/d) peuvent être soumis à l'action d'un agent de thionation, comme le réactif de Lawesson pour conduire au composé de formule (I/e), cas particulier des composés de formule (I): 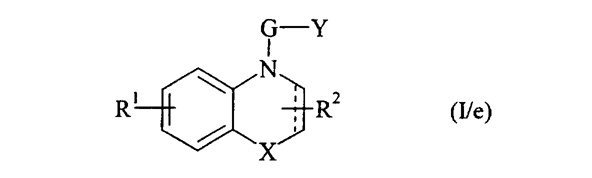 dans laquelle R¹, R², X, G et la représentation ---- sont définis comme précédemment et Y représente un groupement 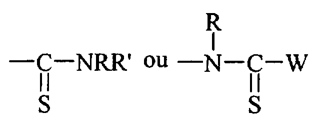 dans lesquels R, R' et W sont tels que définis précédemment,
les composés (I/a) à (I/e) formant l'ensemble des composés de formule (I) et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

12. Compositions pharmaceutiques contenant les produits de formule (I) selon l'une quelconque des revendications 1 à 10 ou un de leurs sels d'addition avec un acide ou une base pharmaceutiquement acceptable en combinaison avec un ou plusieurs excipients pharmaceutiquement acceptables.

13. Compositions pharmaceutiques selon la revendication 12 utiles pour la fabrication de médicaments pour traiter les troubles du système mélatoninergique.

## Claims

1. Compounds of formula (I): 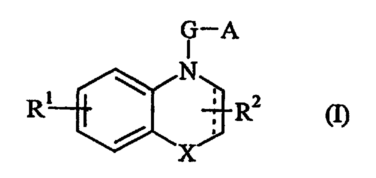 wherein:
◆ R¹ represents a halogen atom or a group R, OR, SR, SO₂NRR', -NRR', 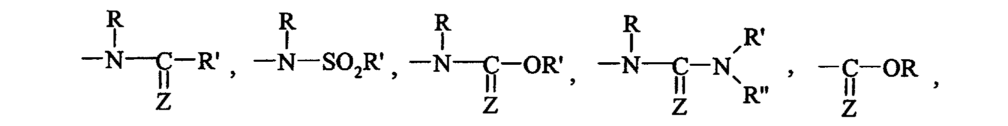 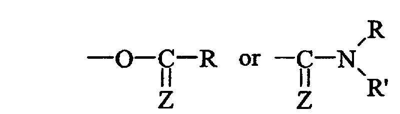 (wherein Z represents a sulphur atom or an oxygen atom and R, R' and R", which are identical or different, each represent a hydrogen atom or an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)-cycloalkyl group, an unsubstituted or substituted (C₃-C₈)cycloalkyl-(C₁-C₆)alkyl group in which the alkyl moiety is linear or branched, an aryl group, an aryl(C₁-C₆)alkyl group in which the alkyl moiety is linear or branched, a heteroaryl group or a heteroaryl- (C₁-C₆)alkyl group in which the alkyl moiety is linear or branched, (R and R') or (R' and R") may together form, with the nitrogen atom carrying them, a morpholinyl, piperidinyl, piperazinyl or pyrrolidinyl group),
◆ G represents a (CH₂)ₙ group in which n is 2 or 3,
◆ A represents a group 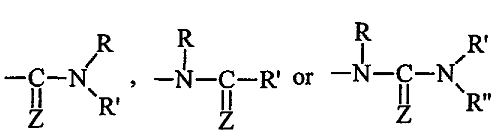 (wherein R, R' R" and Z are as defined hereinbefore),
◆ R² represents a halogen atom or a group R, OR, COR, COOR or OCOR (wherein R is as defined hereinbefore),
◆ X represents an oxygen atom or a sulphur atom,
◆ the symbol ---- denotes that the bond is single or double, the valency of the atoms being respected,
wherein:
- unless specified otherwise, the groups comprising an alkyl moiety in their structure contain from 1 to 6 carbon atoms and may be linear or branched,
- the term "substituted" applied to "alkyl", "alkenyl", "alkynyl" or "cycloalkyl" denotes that those groups may be substituted by one or more identical or different groups selected from hydroxy, alkoxy, alkyl, polyhaloalkyl, amino (unsubstituted or substituted by one or two linear or branched (C₁-C₆)alkyl groups) and halogen atoms,
- the term "substituted" applied to "cycloalkylalkyl" denotes that the cyclic moiety of the group is substituted by one or more identical or different groups selected from hydroxy, alkoxy, alkyl, polyhaloalkyl, amino (unsubstituted or substituted by one or two linear or branched (C₁-C₆)alkyl groups) and halogen atoms,
- "aryl" denotes a phenyl, naphthyl or biphenyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, alkoxycarbonyl, amido and halogen atoms,
- "heteroaryl" denotes any mono- or bi-cyclic aromatic group containing from one to three hetero atoms selected from oxygen, sulphur and nitrogen, those groups being unsubstituted or substituted by one or more identical or different groups selected from hydroxy, alkoxy, alkyl, amino, alkylamino, dialkylamino, nitro, cyano, polyhaloalkyl, formyl, carboxy, alkoxycarbonyl, amido and halogen atoms,
their enantiomers and diastereoisomers, and also addition salts thereof with a pharmaceutically acceptable acid or base.

2. Compounds of formula (I) according to claim 1, wherein X represents an oxygen atom, their enantiomers and diastereoisomers, and also addition salts thereof with a pharmaceutically acceptable acid or base.

3. Compounds of formula (I) according to claim 1 that are (dihydro)benzoxazine compounds, their enantiomers and diastereoisomers, and also addition salts thereof with a pharmaceutically acceptable acid or base.

4. Compounds of formula (I) according to claim 1, wherein R¹ represents an OR group, their enantiomers and diastereoisomers, and also addition salts thereof with a pharmaceutically acceptable acid or base.

5. Compounds of formula (I) according to claim 1, wherein R² represents a hydrogen atom, their enantiomers and diastereoisomers, and also addition salts thereof with a pharmaceutically acceptable acid or base.

6. Compounds of formula (I) according to claim 1, wherein R² represents an unsubstituted or substituted phenyl group, their enantiomers and diastereoisomers, and also addition salts thereof with a pharmaceutically acceptable acid or base.

7. Compounds of formula (I) according to claim 1, wherein A represents a NHCOR group, their enantiomers and diastereoisomers, and also addition salts thereof with a pharmaceutically acceptable acid or base.

8. Compounds of formula (I) according to claim 1, wherein A represents a CONHR group, their enantiomers and diastereoisomers, and also addition salts thereof with a pharmaceutically acceptable acid or base.

9. Compounds of formula (I) according to claim 1 that are *N*-[2-(6-hydroxy-2,3-dihydro-4*H*-1,4-benzoxazin-4-yl)ethyl]acetamide, *N*-[2-(6-methoxy-2,3-dihydro-4*H*-1,4-benzoxazin-4-yl)ethyl]acetamide, *N*-[2-(6-methoxy-2,3-dihydro-4*H*-1,4-benzoxazin-4-yl)ethyl]butanamide, *N*-[2-(6-methoxy-2,3-dihydro-4*H*-1,4-benzoxazin-4-yl)ethyl]-3-butenamide, *N*-[2-(6-methoxy-2,3-dihydro-4*H*-1,4-benzoxazin-4-yl)ethyl]cyclopropanecarboxamide, *N*-[2-(6-methoxy-2,3-dihydro-4*H*-1,4-benzoxazin-4-yl)ethyl]-2-furamide, *N*-[2-(6-methoxy-2,3-dihydro-4*H*-1,4-benzoxazin-4-yl)ethyl]benzamide, *N*-[2-(6-hydroxy-2,3-dihydro-4*H*-1,4-benzoxazin-4-yl)ethyl]-2-furamide, their enantiomers and diastereoisomers, and also addition salts thereof with a pharmaceutically acceptable acid or base.

10. Compounds of formula (I) according to claim 1 that are *N*-[2-(6-methoxy-3-phenyl-2,3-dihydro-4*H*-1,4-benzoxazin-4-yl)ethyl]acetamide, *N*-[2-(6-methoxy-3-phenyl-2,3-dihydro-4*H*-1,4-benzoxazin-4-yl]ethyl)benzamide, *N*-[2-(6-methoxy-3-phenyl-2,3-dihydro-4*H*-1,4-benzoxazin-4-yl)ethyl]-2-furamide, *N*-[2-(6-methoxy-3-phenyl-2,3-dihydro-4*H*-1,4-benzoxazin-4-yl)ethyl]butanamide, *N*-[2-(6-methoxy-2-phenyl-2,3-dihydro-4*H*-1,4-benzoxazin-4-yl)ethyl]acetamide, *N*-[2-(6-methoxy-2-phenyl-2,3-dihydro-4*H*-1,4-benzoxazin-4-yl)ethyl]-2-furamide, *N*-[2-(6-methoxy-2-phenyl-2,3-dihydro-4*H*-1,4-benzoxazin-4-yl)ethyl]benzamide, *N*-[2-(6-methoxy-2-phenyl-2,3-dihydro-4*H*-1,4-benzoxazin-4-yl)ethyl]butanamide, *N*-[2-(6-methoxy-2-phenyl-2,3-dihydro-4*H*-1,4-benzoxazin-4-yl)ethyl]cyclopropanecarboxamide, their enantiomers and diastereoisomers, and also addition salts thereof with a pharmaceutically acceptable acid or base.

11. Process for the preparation of the compounds of formula (I) according to claim 1, **characterised in that** there is used as starting material a compound of formula (II): 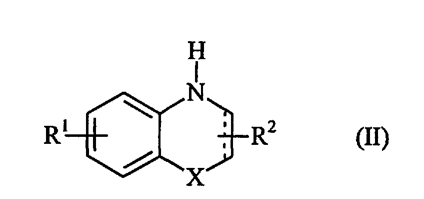 wherein R¹, R², X and the symbol ---- are as defined in claim 1,
with which there is reacted, in basic medium, a compound of formula (III):
Br-G-CN (III)
wherein G is as defined in claim 1,
to yield a compound of formula (IV) : 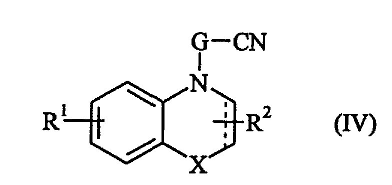 wherein R¹, R², X, G and the symbol ---- are as defined hereinbefore,
which is subjected to hydrolysis, under acid or basic conditions, to yield a compound of formula (V): 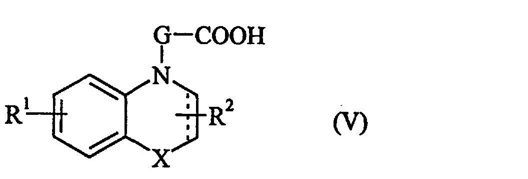 wherein R¹, R², X, G and the symbol ---- have the same definitions as hereinbefore,
which is acted upon, in the presence of a coupling agent or after conversion into the corresponding acid chloride, by an amine HNRR' wherein R and R' are as defined in claim 1, to yield a compound of formula (I/a), a particular case of the compounds of formula (I): 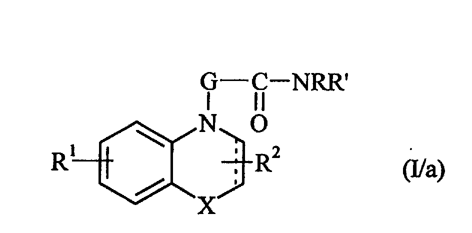 wherein R¹, R², X, G, R, R' and the symbol ---- are as defined hereinbefore,
which compound of formula (I/a) is subjected, when R and R' simultaneously represent a hydrogen atom, to the action of NaOBr to yield, after hydrolysis, a compound of formula (VI): 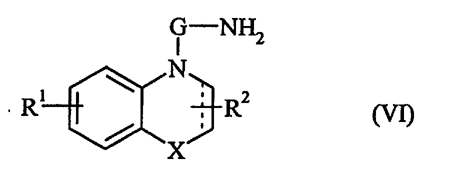 wherein R¹, R², X, G and the symbol ---- are as defined hereinbefore,
which compounds of formula (VI) (which furthermore can be obtained by reduction of the compound of formula (IV)) are subjected to the action:
- of an acyl chloride of formula (VII): 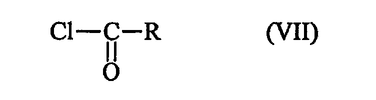 wherein R is as defined hereinbefore, or to a corresponding (mixed or symmetrical) acid anhydride,
to obtain a compound of formula (I/b), a particular case of the compounds of formula (I): 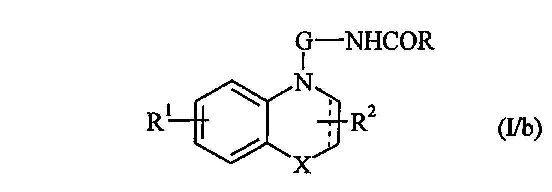 wherein R¹, R², R, X, G and the symbol ---- have the same definitions as hereinbefore,
- or of a compound of formula (VIII):
O = C = N - R (VIII)
wherein R is as defined hereinbefore,
to yield a compound of formula (I/c), a particular case of the compounds of formula (I): 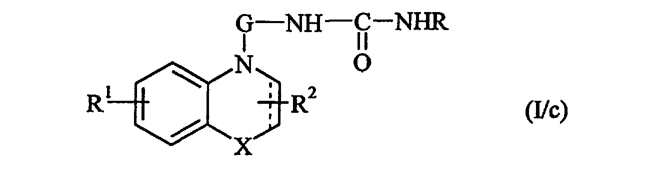 wherein R¹, R², R, X, G and the symbol ---- are as defined hereinbefore,
wherein the compounds of formulae (I/b) and (I/c) may be subjected to the action of a compound of formula (IX):
Rₐ - J (IX)
wherein Rₐ may have any of the meanings of R with the exception of a hydrogen atom and J represents a leaving group, such as a halogen atom or a tosyl group,
to yield a compound of formula (I/d), a particular case of the compounds of formula (I) : 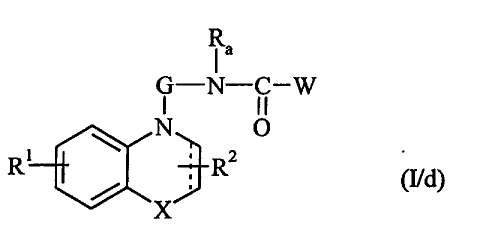 wherein R¹, R², X, G, Rₐ and the symbol ---- have the same definitions as hereinbefore and W represents a group R or -NRR' wherein R and R' are as defined hereinbefore,
and/or the compounds of formulae (I/a), (I/b), (I/c) and (I/d) may be subjected to the action of a thionisation agent, such as Lawesson's reagent, to yield a compound of formula (I/e), a particular case of the compounds of formula (I): 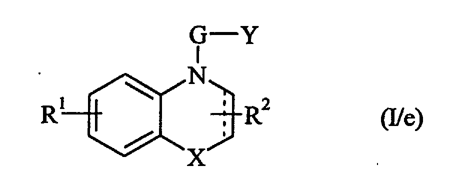 wherein R¹, R², X, G and the symbol ---- are as defined hereinbefore and Y represents a group 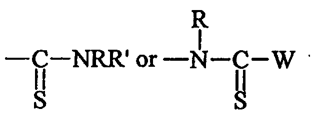 wherein R, R' and W are as defined hereinbefore,
the compounds (I/a) to (I/e) constituting the totality of the compounds of formula (I) and wherein those compounds may be purified according to a conventional separation technique, are converted, if desired, into their addition salts with a pharmaceutically acceptable acid or base, and are optionally separated into their isomers according to a conventional separation technique.

12. Pharmaceutical compositions comprising the compounds of formula (I) according to any one of claims 1 to 10, or an addition salt thereof with a pharmaceutically acceptable acid or base, in combination with one or more pharmaceuticallly acceptable excipients.

13. Pharmaceutical compositions according to claim 12, for use in the preparation of medicaments for the treatment of disorders of the melatoninergic system.

## Patentansprüche

1. Verbindungen der Formel (I): 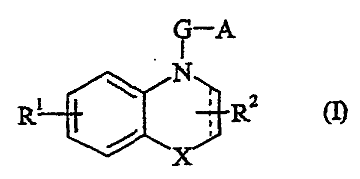 in der:
◆ R¹ ein Halogenatom oder eine Gruppe R, OR, SR, SO₂NRR', -NRR', 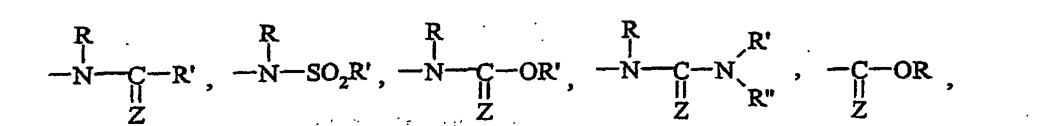 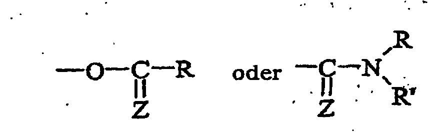 (worin Z ein Schwefelatom oder Sauerstoffatom und R, R' und R", die gleichartig oder verschieden sind, Wasserstoffatome oder substituierte oder nichtsubstituierte, geradkettige oder verzweigte (C₁-C₆)-Alkylgruppen, substituierte oder nichtsubstituierte, geradkettige oder verzweigte (C₂-C₆)-Alkenylgruppen, substituierte oder nichtsubstituierte, geradkettige oder verzweigte (C₂-C₆)-Alkinylgruppen, substituierte oder nichtsubstituierte (C₃-C₈)-Cycloalkylgruppen, substituierte oder nichtsubstituierte, geradkettige oder verzweigte (C₃-C₈)-Cycloalkyl-(C₁-C₆)-alkylgruppen, Arylgruppen, geradkettige oder verzweigte Aryl-(C₁-C₆)-Alkylgruppen, Heteroarylgruppen oder geradkettige oder verzweigte Heteroaryl-(C₁-C₆)-alkylgruppen darstellen,
wobei (R und R') oder (R' und R") gemeinsam mit dem sie tragenden Stickstoffatom eine Morpholinyl-, Piperidinyl-, Piperazinyl- oder Pirrolidinylgruppe bilden können), bedeutet,
◆ G eine Gruppe (CH₂)ₙ, worin n den Wert oder 2 oder 3 besitzt, darstellt,
◆ A eine Gruppe 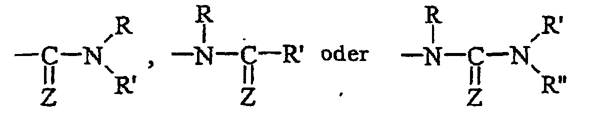 (worin R, R', R" und Z die oben angegebenen Bedeutungen besitzen) bedeutet,
◆ R² ein Halogenatom oder eine Gruppe R, OR, COR, COOR oder OCOR (worin R die oben angegebenen Bedeutungen besitzt) darstellt,
◆ X ein Sauerstoffatom oder ein Schwefelatom bedeutet und
◆ das Symbol ---- bedeutet, daß die Bindung einfach oder doppelt ist, wobei es sich versteht, daß die Wertigkeit der Atome berücksichtigt wird,
mit der Maßgabe, daß:
- wenn nichts anderes angegeben ist, die einen Alkylrest in ihrer Struktur enthaltenden Gruppen 1 bis 6 Kohlenstoffatome aufweisen und geradkettig oder verzweigt sein können,
- der Begriff «substituiert», unter Bezug auf die Begriffe «Alkyl», «Alkenyl», «Alkinyl» oder «Cycloalkyl» bedeutet, daß diese Gruppen durch eine oder mehrere, gleichartige oder verschiedene Gruppen ausgewählt aus Hydroxy, Alkoxy, Alkyl, Polyhalogenalkyl, Amino (nichtsubstituiert oder durch eine oder zwei geradkettige oder verzweigte (C₁-C₆)-Alkylgruppen substituiert) und Halogenatome substituiert sein können,
- der Begriff «substituiert», unter Bezug auf den Begriff «Cycloalkylalkyl», bedeutet, daß der cyclische Teil der Gruppe durch eine oder mehrere, gleichartige oder verschiedene Gruppen substituiert ist ausgewählt aus Hydroxy, Alkoxy, Alkyl, Polyhalogenalkyl, Amino (nichtsubstituiert oder durch eine oder zwei geradkettige oder verzweigte (C₁-C₆)-Alkylgruppen substituiert) und Halogenatomen substituiert ist,
- unter «Aryl» eine Phenyl-, Naphthyl- oder Biphenylgruppe zu verstehen ist, wobei diese Gruppen nichtsubstituiert oder durch eine oder mehrere gleichartige oder verschiedene Gruppen substituiert sind ausgewählt aus Hydroxy, Alkoxy, Alkyl, Amino, Alkylamino, Dialkylamino, Nitro, Cyano, Polyhalogenalkyl, Formyl, Carboxy, Alkyloxycarbonyl. Amido und Halogenatomen,
- unter «Heteroaryl» man jede aromatische, mono- oder bicyclische Gruppe versteht, die eins bis drei Heteroatome ausgewählt aus Sauerstoff, Schwefel und Stickstoff aufweist, wobei diese Gruppen nichtsubstituiert sind oder substituiert durch eine oder mehrere gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Alkyloxycarbonyl, Amido und Halogenatomen,
deren Enantiomere und Diastereoisomere sowie deren Additionssalze mit einer pharmazeutisch annehmbaren Säure oder Base.

2. Verbindungen der Formel (I) nach Anspruch 1, worin X ein Sauerstoffatom darstellt, deren Enantiomere und Diastereoisomere sowie deren Additionssalze mit einer pharmazeutisch annehmbaren Säure oder Base.

3. Verbindungen der Formel (I) nach Anspruch 1, nämlich (Dihydro)-benzoxazin-Derivate, deren Enantiomere und Diastereoisomere sowie deren Additionssalze mit einer pharmazeutisch annehmbaren Säure oder Base.

4. Verbindungen der Formel (I) nach Anspruch 1, worin R¹ eine Gruppe OR darstellt, deren Enantiomere und Diastereoisomere sowie deren Additionssalze mit einer pharmazeutisch annehmbaren Säure oder Base.

5. Verbindungen der Formel (I) nach Anspruch 1, worin R² ein Wasserstoffatom darstellt, deren Enantiomere und Diastereoisomere sowie deren Additionssalze mit einer pharmazeutisch annehmbaren Säure oder Base.

6. Verbindungen der Formel (I) nach Anspruch 1, worin R² eine gegebenenfalls substituierte Phenylgruppe darstellt, deren Enantiomere und Diastereoisomere sowie deren Additionssalze mit einer pharmazeutisch annehmbaren Säure oder Base.

7. Verbindungen der Formel (I) nach Anspruch 1, worin A eine Gruppe NHCOR darstellt, deren Enantiomere und Diastereoisomere sowie deren Additionssalze mit einer pharmazeutisch annehmbaren Säure oder Base.

8. Verbindungen der Formel (I) nach Anspruch 1, A eine Gruppe CONHR darstellt, deren Enantiomere und Diastereoisomere sowie deren Additionssalze mit einer pharmazeutisch annehmbaren Säure oder Base.

9. Verbindungen der Formel (I) nach Anspruch 1, nämlich
*N*-[2-(6-Hyddroxy-2,3-dihydro-4*H*-1,4-benzoxazin-4-yl)-ethyl]-acetamid,
*N-*[2-(6-Methoxy-2,3-dihydro-4*H-*1,4-benzoxazin-4-yl)-ethyl]-acetamid,
*N*-[2-(6-Methoxy-2,3-dihydro-4*H*-1,4-benzoxazin-4-yl)-ethyl]-butanamid,
*N*-[2-(6-Methoxy-2,3-dihydro-4*H*-1,4-benzoxazin-4-yl)-ethyl]-3-butenamid,
*N*-[2-(6-Methoxy-2,3-dihydro-4*H*-1,4-benzoxazin-4-yl)-ethyl]-cyclopropancarboxamid,
*N*-[2-(6-Methoxy-2,3-dihydro-4*H*-1,4-benzoxazin-4-yl)-ethyl]-2-furancarbonsäureamid,
*N*-[2-(6-Methoxy-2,3-dihydro-4*H*-1,4-benzoxazin-4-yl)-ethyl]-benzamid,
*N*-[2-(6-Hydroxy-2,3-dihydro-4*H*-1,4-benzoxazin-4-yl)-ethyl]-2-furancarbonsäureamid,
deren Enantiomere und Diastereoisomere sowie deren Additionssalze mit einer pharmazeutisch annehmbaren Säure oder Base.

10. Verbindungen der Formel (I) nach Anspruch 1, nämlich
*N*-[2-(6-Methoxy-3-phenyl-2,3-dihydro-4*H*-1,4-benzoxazin-4-yl)-ethyl]-acetamid.
*N*-[2-(6-Methoxy-3-phenyl-2,3-dlhydro-4*H*-1,4-benzoxazin-4-yl)-ethyl]-benzamid,
*N*-[2-(6-Methoxy-3-phenyl-2,3-dihydro-4*H*-1,4-benzoxazin-4-yl)-ethyl]-2-furancar bonsäureamid,
*N*-(2-(6-Methoxy-3-phenyl-2,3-dihydro-4*H*-1,4-benzoxazin-4-yl)-ethyl]-butanamid,
*N*-[2-(6-Methoxy-2-phenyl-2,3-dihydro-4H-1,4-benzoxazin-4-yl)-ethyl]acetamid,
*N*-[2-(6-Methoxy-2-phenyl-2,3-dihydro-4*H*-1,4-benzoxazin-4-yl)-ethyl]-2-furancar bonsäureamid.
*N*-[2-(6-Methoxy-2-phenyl-2,3-dihydro-4*H*-1,4-benzoxazin-4-yl)-ethyl]-benzamid,
*N*-[2-(6-Methoxy-2-phenyl-2,3-dihydro-4*H*-1,4-benzoxazin-4-yI)-ethyl]-butanamid,
*N*-[2-(6-Methoxy-2-phenyl-2,3-dihydro-4*H*-1,4-benzoxazin-4-yl)-ethyl]-cyclopropancarboxamid,
deren Enantiomeren und Diastereoisomeren sowie deren Additionssalze mit einer pharmazeutisch annehmbaren Säure oder Base.

11. Verfahren zur Herstellung der Verbindungen der Formel (I) nach Anspruch 1, **dadurch gekennzeichnet, daß** man als Ausgangsprodukt die Verbindung der Formel (II) verwendet: 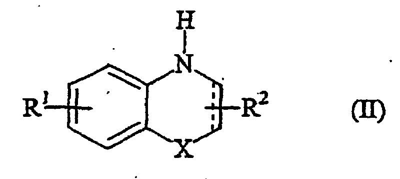 in der R¹, R², X und das Symbol ---- die in Anspruch 1 angegebenen Bedeutungen besitzen,
welche man in basischem Medium mit der Verbindung der Formel (III) umsetzt:
Br - G - CN (III)
in der G die in Anspruch 1 angegebenen Bedeutungen besitzt,
zur Bildung der Verbindung der Formel (IV): 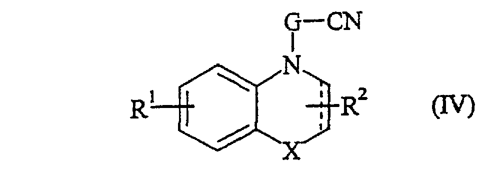 in der R¹, R², X, G und das Symbol ---- die oben angegebenen Bedeutungen besitzen,
welche man einer Hydrolyse unter sauren oder basischen Bedingungen unterwirft zur Bildung der Verbindung der Formel (V): 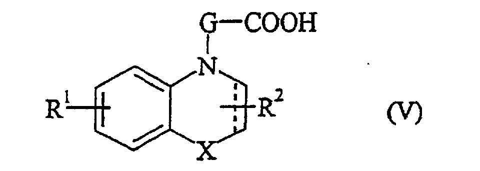 in der R¹, R², X, G und das Symbol ---- die oben angegebenen Bedeutungen besitzen,
welche man in Gegenwart eines Kupplungsmittels oder nach der Umwandlung in das entsprechende Säurechlorid mit einem Amin HNRR', worin R und R' di ein Anspruch 1 angegebenen Bedeutungen besitzen, umsetzt zur Bildung der Verbindung der Formel (I/a), einem Sonderfall der Verbindungen der Formel (I): 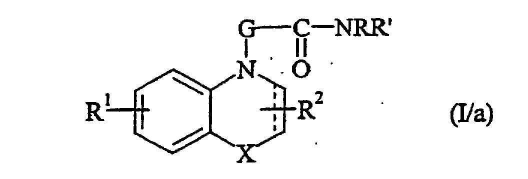 in der R¹, R², X, G, R, R' und das Symbol ---- die oben angegebenen Bedeutungen besitzen,
welche Verbindung der Formel (I/a) man dann, wenn R und R' gleichzeitig ein Wasserstoffatom bedeuten, der Einwirkung von NaOBr unterwirft, so daß man nach der Hydrolyse die Verbindung der Formel (VI) erhält: 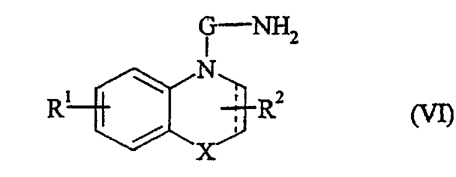 in der R¹, R², X, G und das Symbol ---- die oben angegebenen Bedeutungen besitzen,
welche Verbindungen der Formel (VI) (welche man auch durch Reduktion der Verbindung der Formel (IV) erhalten kann) man der Einwirkung:
- eines Acylchlorids der Formel (VII): 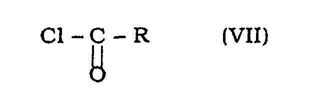 worin R die oben angegebenen Bedeutungen besitzt, oder des entsprechenden (gemischten oder symmetrischen) Säureanhydrids unterwirft
zur Bildung der Verbindung der Formel (I/b), einem Sonderfall der Verbindungen der Formel (I): 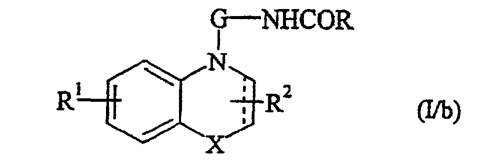 in der R¹, R², R, X, G und das Symbol ---- die oben angegebenen Bedeutungen besitzen,
- oder einer Verbindung der Formel (VIII):
O = C = N - R (VIII)
in der R die oben angegebenen Bedeutungen besitzt,
unterwirft zur Bildung der Verbindung der Formel (I/c), einem Sonderfall der Verbindungen der Formel (I): 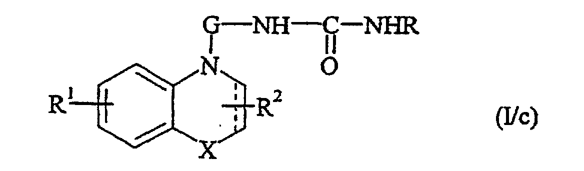 in der R¹, R², R, X, G und das Symbol ---- die oben angegebenen Bedeutungen besitzen,
welche Verbindungen der Formeln (I/b) und (I/c) man der Einwirkung einer Verbindung der Formel (IX):
Rₐ - J (IX)
in der Rₐ sämtliche Bedeutungen von R mit Ausnahme des Wasserstoffatoms besitzt und J eine austretende Gruppe, wie ein Halogenatom oder eine Tosylgruppe darstellt,
unterwerfen kann zur Bildung der Verbindung der Formel (I/d), einem Sonderfall der Verbindungen der Formel (I): 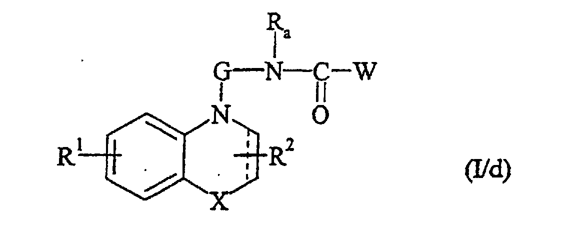 in der R¹, R², X, G, Rₐ und das Symbol ---- die oben angegebenen Bedeutungen besitzen und W eine Gruppe R oder -NRR' darstellt, worin R und R' die oben angegebenen Bedeutungen besitzen,
und/oder man die Verbindungen der Formeln (I/a), (I/b), (I/c) und (I/d) man der Einwirkung eines Thionierungsmittels, wie des Lawesson-Reagens, unterwerfen kann zur Bildung der Verbindung der Formel (I/e), einem Sonderfall der Verbindungen der Formel (I): 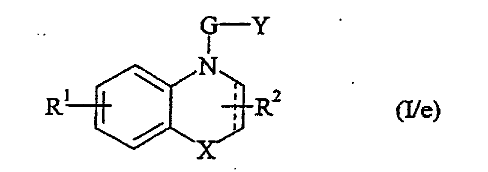 in der R¹, R², X, G und das Symbol ---- die oben angegebenen Bedeutungen besitzen und Y eine Gruppe 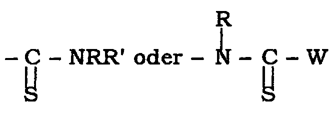 darstellt, worin R, R' und W die oben angegebenen Bedeutungen besitzen,
welche Verbindungen (I/a) bis (I/e) die Gesamtheit der Verbindungen der Formel (I) bilden und mit Hilfe einer klassischen Reinigungsmethode gereinigt werden können, man gewünschtenfalls in ihre Additionssalze mit einer pharmazeutisch annehmbaren Säure oder Base umgewandelt werden können und die man gegebenenfalls mit Hilfe einer klassischen Trennmethode in ihre Isomeren auftrennen kann.

12. Pharmazeutische Zubereitungen enthaltend die Produkte der Formel (I) nach irgendeinem der Ansprüche 1 bis 10 oder eines ihrer Additionssalze mit einer pharmazeutisch annehmbaren Säure oder Base in Kombination mit einem oder mehreren, pharmazeutisch annehmbaren Trägermaterialien oder Bindemitteln.

13. Pharmazeutische Zubereitungen nach Anspruch 12 zur Herstellung von Arzneimitteln zur Behandlung von Störungen des melatoninergischen Systems.
